# FASCICULE DE BREVET EUROPEEN

(11) **EP 1 728 074 B1**
(45) Date de publication et mention de la délivrance du brevet: **21.11.2007**
(21) Numéro de dépôt: 04767658.0
(22) Date de dépôt: 12.07.2004
(51) Int. Cl.: G01N 33/18, C12Q 1/68

(54) **PROCEDE D'EXTRACTION DE COMPOSES OU COMPLEXES INTRACELLULAIRES A PARTIR D'UN ECHANTILLON CONTENANT DES CELLULES EN SUSPENSION DANS UN LIQUIDE**
VERFAHREN ZUR EXTRAKTION VON INTRAZELLULÄREN VERBINDUNGEN ODER KOMPLEXEN AUSGEHEND VON EINER PROBE DIE EINE FLÜSSIGE ZELLSUSPENSION BEINHALTET
METHOD FOR EXTRACTING INTRACELLULAR COMPLEXES OR COMPOUNDS FROM A SAMPLE CONTAINING CELLS IN A LIQUID SUSPENSION

(30) Priorité: 26.03.2004 FR 0403142
(43) Date de publication de la demande: 06.12.2006
(73) Titulaire: SOCIETE D'AMENAGEMENT URBAIN ET RURAL, 78280 Guyancourt (FR)
(72) Inventeur: GRESLE, Anne, F-78180 Montigny-Le-Bretonneux (FR)
(74) Mandataire: Ahner, Francis
(86) Numéro de dépôt international: PCT/FR2004/001827
(87) Numéro de publication internationale: WO 2005/103682

(56) Documents cités:
- WO-A-01/19963
- FR-A- 2 827 873
- DATTA A R ET AL: "DETECTION OF HEMOLYTIC LISTERIA-MONOCYTOGENES BY USING DNA COLONY HYBRIDIZATION" APPLIED AND ENVIRONMENTAL MICROBIOLOGY, vol. 53, no. 9, 1987, pages 2256-2259, XP009040454 ISSN: 0099-2240
- JONES C L ET AL: "AN OLIGONUCLEOTIDE PROBE TO ASSAY LYSIS AND DNA HYBRIDIZATION OF A DIVERSE SET OF BACTERIA" ANALYTICAL BIOCHEMISTRY, ACADEMIC PRESS, SAN DIEGO, CA, US, vol. 181, janvier 1989 (1989-01), pages 23-27, XP000212725 ISSN: 0003-2697
- FUJIKAWA H ET AL: "KINETICS OF ESCHERICHIA COLI DESTRUCTION BY MICROWAVE IRRADIATION" APPLIED AND ENVIRONMENTAL MICROBIOLOGY, WASHINGTON,DC, US, vol. 58, no. 3, mars 1992 (1992-03), pages 920-924, XP000989723 ISSN: 0099-2240 cité dans la demande
- LIU CHUANBIN ET AL: "Trehalose extraction from Saccharomyces cerevisiae after microwave treatment" BIOTECHNOLOGY TECHNIQUES, vol. 12, no. 12, décembre 1998 (1998-12), pages 941-943, XP009040046 ISSN: 0951-208X

## Description

La présente invention concerne un procédé d'extraction de composés ou complexes intracellulaires, tels que des acides nucléiques, à partir d'un échantillon contenant des cellules en suspension dans un liquide. Le procédé selon la présente invention peut avantageusement être appliqué à la détection de la présence d'organismes pathogènes dans un échantillon liquide. Les procédés selon la présente invention peuvent être avantageusement entièrement automatisés. L'invention a également pour objet un dispositif d'extraction de composés ou complexes intracellulaires, tels que des acides nucléiques, de cellules en suspension dans un échantillon liquide, ainsi qu'un dispositif de détection de la présence d'organismes pathogènes dans un échantillon liquide, convenant à la mise en oeuvre desdits procédés.

La lyse cellulaire est une étape nécessaire pour extraire des composés ou complexes intracellulaires, tels que des acides nucléiques, de cellules en suspension dans un échantillon liquide, tel que l'eau.

La lyse cellulaire consiste en une rupture ou une ouverture de cellules visant à libérer leur contenu, et notamment les acides nucléiques présents dans ces cellules. Les conditions opératoires de cette lyse doivent être adaptées de manière à éviter toute altération du contenu desdites cellules.

La lyse des cellules est en général réalisée à l'aide d'agents chimiques tels que des détergents, des solvants, des agents alcalins de digestion ou des enzymes telles que des protéinases, qui permettent de digérer les parois cellulaires. Cette technique de lyse à l'aide d'agents chimiques présente divers inconvénients, tels que la nécessité de s'approvisionner et de stocker les réactifs chimiques appropriés.

La lyse des cellules peut également être réalisée thermiquement à l'aide de plaques chauffantes, permettant de chauffer l'échantillon liquide contenant des cellules en suspension. Cette technique s'avère néanmoins très longue et peut engendrer une évaporation trop forte de l'échantillon, ainsi que la dégradation du contenu des cellules lysées.

La lyse cellulaire peut également être réalisée par ultrasons, par agitation ou par vibration mécanique, par chocs osmotiques ou encore par broyage sur perles agitées. Cependant, si ces différentes techniques s'avèrent efficaces, elles peuvent conduire à une dégradation de la structure chimique du contenu des cellules, et en particulier des acides nucléiques présents dans ces cellules.

La lyse cellulaire peut également être réalisée par micro-ondes. L'utilisation de micro-ondes permet d'obtenir un craquage rapide des cellules. La demande WO 01/19963 décrit ainsi le chauffage de petits échantillons liquides, de volumes inférieurs à 1 ml, à l'aide de micro-ondes, dont la fréquence de radiation est comprise entre 18 et 26 GHz. La lyse est alors obtenue par élévation de température, de la même façon que si on réalisait le chauffage de l'échantillon par une méthode classique (Cf. Hiroshi Fujikawa, « Kinetics of Escherichia coli destruction by microwave irradiation », Applied and Environmental Microbiology, 1992, p. 920-924). Le système décrit dans WO 01/19963 présente plusieurs inconvénients. La lyse cellulaire est effectuée directement sur les échantillons liquides, ce qui nécessite par conséquent une forte énergie et un temps relativement long pour chauffer les échantillons. En outre, le chauffage par micro-ondes de cellules en suspension dans des échantillons liquides peut engendrer des dégradations, voire des destructions, de la structure chimique du contenu des cellules, et en particulier des acides nucléiques présents dans ces cellules.

Il existait ainsi un besoin de mettre au point un procédé ainsi qu'un dispositif d'extraction de composés ou complexes intracellulaires, tels que des acides nucléiques, à partir d'un échantillon contenant des cellules en suspension dans un liquide, lesdits procédé et dispositif comprenant une étape de lyse cellulaire et ne présentant pas les inconvénients des techniques de l'art antérieur. Il existait ainsi un besoin de mettre au point un procédé ainsi qu'un dispositif permettant d'effectuer de manière efficace et rapide une lyse des cellules à l'aide d'un dispositif très simple, à moindre coût, nécessitant peu d'énergie, permettant de respecter totalement l'intégrité du contenu des cellules et de récupérer ensuite lesdits composés ou complexes intracellulaires extraits des cellules lysées avec un rendement élevé.

La présente invention vient combler ce besoin. La demanderesse a ainsi découvert de manière surprenante que la lyse des cellules pouvait être réalisée par micro-ondes, en procédant préalablement à une fixation des cellules sur un support solide, et à un séchage des cellules fixées afin d'éliminer le liquide extracellulaire. Cette élimination du liquide extracellulaire permet ainsi de chauffer directement le milieu intracellulaire par une absorption sélective des micro-ondes dans les cellules. Il en résulte alors une lyse cellulaire rapide et efficace à l'aide d'une très faible quantité d'énergie, tout en évitant une altération du contenu des cellules. Les composés ou complexes intracellulaires, tels que les acides nucléiques, ainsi libérés peuvent ensuite être fixés directement sur ledit support solide ou sur un autre support solide, sans être dilués, permettant la purification desdits composés ou complexes intracellulaires in situ et leur récupération ultérieure par élution à l'aide d'un solvant.

La demanderesse a également découvert de manière surprenante que le procédé d'extraction selon la présente invention ne nécessitait pas de réactifs chimiques pour effectuer la lyse des cellules pouvant par la suite perturber l'aval du procédé, qu'un tel procédé pouvait être entièrement automatisable et qu'il pouvait être avantageusement utilisé pour la détection de la présence d'organismes pathogènes dans un échantillon liquide.

La présente invention a ainsi pour objet un procédé d'extraction de composés ou complexes intracellulaires, tels que des acides nucléiques, à partir d'un échantillon contenant des cellules en suspension dans un liquide, caractérisé en ce qu'il comprend les étapes successives suivantes :
- fixation des cellules sur un support solide,
- séchage des cellules fixées sur ledit support,
- lyse des cellules sèches par micro-ondes, puis
- récupération desdits composés ou complexes intracellulaires.

Les composés ou complexes intracellulaires peuvent être tout composé biologique tel que des protéines, notamment des protéines recombinantes, non sécrétées, ou des acides nucléiques, ou encore des organites.

Avantageusement selon la présente invention, le liquide est de l'eau ou est un liquide constitué essentiellement d'eau. Ainsi, le liquide peut par exemple être du lait ou un jus de fruits. Lorsque le liquide est de l'eau, l'eau peut être issue des réseaux d'eau potable, des réseaux d'eau industrielle ou d'eau usée, ou encore des réseaux d'eau de refroidissement.

Selon une caractéristique particulière de la présente invention, le volume initial d'échantillon est de l'ordre de 10 ml à 1 litre, de préférence de 10 ml à 200 ml, encore plus préférentiellement de 30 ml à 100 ml, encore plus préférentiellement de l'ordre de 40 à 50 ml.

La fixation des cellules sur un support solide permet de séparer par filtration les cellules du liquide et de fixer ainsi les cellules avant de procéder au séchage des cellules, puis à la lyse desdites cellules afin d'extraire les composés ou complexes intracellulaires, tels que les acides nucléiques, des cellules lysées.

Avantageusement selon la présente invention, le liquide à filtrer est pompé sous pression (par exemple 2 bars) au travers du support solide qui contient typiquement un matériau filtrant et permet de retenir et piéger les cellules sur le support. Le liquide est quant à lui récupéré après filtration et est avantageusement dirigé vers une évacuation de déchets.

Selon un mode de réalisation particulier de la présente invention, la fixation des cellules est réalisée par adsorption des cellules sur le support solide.
Avantageusement selon la présente invention, le support solide contient au moins un matériau poreux, avantageusement adsorbant.

De manière avantageuse selon la présente invention, le support solide contient un matériau poreux adsorbant, avantageusement un matériau vitreux ou un composé à base de silice, tel que du verre, des fibres de verre, un oxyde de silice ou un silicate. La nature du matériau est choisie de manière à fixer les cellules et à les piéger sur toute la surface du matériau poreux.

Le support solide peut également contenir une matrice poreuse, dont la taille des pores est de l'ordre de grandeur de la taille des cellules à lyser ou est inférieure à la taille des cellules à lyser, permettant de retenir ainsi sur le support lesdites cellules.

Dans le cadre de la présente invention, la fixation des cellules peut également être réalisée par tout autre moyen connu de l'Homme du métier.

Avantageusement, le procédé selon la présente invention comprend en outre une étape préalable de concentration des cellules, avant de procéder à l'étape de fixation des cellules sur le support solide. La concentration des cellules peut être réalisée par précipitation, co-précipitation ou précipitation par inclusion, ou par tout autre technique connue de l'homme du métier telle que la centrifugation. La concentration des cellules par précipitation, co-précipitation ou précipitation par inclusion peut être effectuée à l'aide de carbonates d'ions bivalents, tels que le carbonate de calcium, le carbonate de strontium et le carbonate de baryum, et leurs mélanges.

Dans un mode de réalisation particulier de la présente invention, le séchage des cellules est réalisé par injection d'air, avantageusement d'air comprimé, ou par injection de tout autre gaz de séchage tel que l'azote, sur le support solide ou au travers dudit support, ou encore par séchage sous vide, en appliquant par exemple un vide sous le support.

Cette étape de séchage des cellules fixées sur le support permet d'éliminer le liquide extracellulaire, et permet par conséquent d'améliorer significativement l'efficacité de l'étape suivante, à savoir la lyse des cellules par micro-ondes, dans la mesure où l'énergie apportée par les micro-ondes permet de chauffer directement le liquide intracellulaire, et non le liquide externe aux cellules. En outre, cette étape de séchage permettant d'éliminer le liquide extracellulaire permet d'éviter les altérations de la structure chimique du contenu des cellules, et en particulier des acides nucléiques présents dans ces cellules.

Par le terme "d'élimination du liquide extracellulaire", on entend au sens de la présente invention une élimination substantielle du liquide extracellulaire. Ainsi, des traces mineures de liquide extracellulaire peuvent subsister à l'issue de cette étape.

Selon une caractéristique particulière de la présente invention, la lyse des cellules par micro-ondes est effectuée à une fréquence de radiation de 1 à 17 GHz, avantageusement de 1 à 15 GHz, encore plus avantageusement de 2 à 10 GHz, et encore plus avantageusement de 2,5 à 5 GHz.

Le procédé selon la présente invention présente l'avantage de minimiser l'énergie à apporter pour lyser les cellules grâce au séchage préalable, ce qui permet de minimiser le risque d'altération des acides nucléiques. L'étape de séchage, avantageusement réalisée par injection d'air sur le support solide ou au travers dudit support, permet ainsi d'évacuer substantiellement le liquide présent dans les pores du support. La lyse des cellules par micro-ondes peut alors être ensuite réalisée en introduisant par exemple le support solide sur lequel sont piégées les cellules dans un four à mscrc-ondes.

La récupération des composés ou complexes intracellulaires peut se faire par tout moyen connu de l'Homme du métier. Avantageusement selon la présente invention, la récupération des composés ou complexes intracellulaires comprend la fixation desdits composés ou complexes intracellulaires sur un support solide, de préférence suivie d'une élution desdits composés ou complexes intracellulaires. Ledit support solide contient avantageusement au moins un matériau poreux, de préférence adsorbant. De manière encore plus avantageuse selon la présente invention, la fixation desdits composés ou complexes intracellulaires est réalisée par adsorption desdits composés ou complexes intracellulaires sur le support solide.

De manière avantageuse selon la présente invention, les composés ou complexes intracellulaires extraits hors des cellules lysées viennent se fixer naturellement sur ledit support, avantageusement par adsorption. Les cellules lysées restent quant à elles sur le support. Un simple lavage du support avec un solvant approprié tel que l'eau permet ensuite de récupérer par élution lesdits composés ou complexes intracellulaires extraits des cellules lysées. Avant de procéder à l'élution des composés ou complexes intracellulaires par lavage du support, lesdits composés ou complexes intracellulaires fixés sur le support peuvent être purifiés à l'aide d'un solvant particulier tel que l'éthanol.

Dans un mode de réalisation particulier de la présente invention, le support solide pour la fixation des cellules et le support solide pour la fixation des composés ou complexes intracellulaires sont confondus.

Ce mode de réalisation s'avère particulièrement avantageux dans la mesure où l'ensemble des étapes peut alors être réalisé sur un même support solide. Un tel mode de réalisation permet ainsi l'utilisation d'un dispositif plus simple et plus compact que les dispositifs généralement utilisés pour extraire les acides nucléiques de cellules, et engendre ainsi une réduction de coût, puisque la fixation des cellules, la lyse des cellules et la fixation des acides nucléiques peuvent être réalisées au sein d'une même unité. Par ailleurs, un tel mode de réalisation est particulièrement avantageux, dans la mesure où l'ensemble des étapes du procédé peut être facilement automatisé.

Avantageusement, le procédé selon la présente invention est un procédé d'extraction d'acides nucléiques de cellules en suspension dans un échantillon liquide comprenant les étapes successives suivantes :
- adsorption des cellules sur un support solide,
- séchage des cellules adsorbées sur ledit support,
- lyse des cellules sèches par micro-ondes,
- adsorption des acides nucléiques ainsi libérés sur ledit support, puis
- élution desdits acides nucléiques.

Avantageusement, le procédé d'extraction d'acides nucléiques selon la présente invention comprend en outre au moins une étape préalable de concentration des cellules.

Tandis que dans le domaine médical, il s'agit d'analyser des échantillons de sang de quelques microlitres à quelques millilitres, dans la recherche et le contrôle portant sur l'environnement, des échantillons de volumes pouvant aller jusqu'à plusieurs dizaines de litres sont usuellement analysés. Cependant, les techniques directes d'ouverture des cellules des organismes ne sont pas réalisables sur des volumes aussi importants, et les cellules doivent ainsi être concentrées dans des volumes de liquide inférieurs à 200 ml, voire inférieurs à 100 ml, avant de pouvoir démarrer la suite du traitement. La difficulté de la concentration de cellules issues d'échantillons de volumes de l'ordre du litre est de perdre le moins possible de cellules, tout en réduisant le volume de l'échantillon jusqu'à un volume de l'ordre de 200 ml ou d'une centaine de ml.

Dans un mode de réalisation particulier de la présente invention, la concentration des cellules est réalisée par précipitation, co-précipitation ou précipitation par inclusion, et/ou la concentration des cellules est réalisée par filtration et/ou à l'aide d'un système immuno-magnétique. Avantageusement selon là présente invention, la précipitation, la co-précipitation ou la précipitation par inclusion est très efficace en terme de piégeage des cellules et/ou organismes, et s'avère très économique.

Avantageusement selon la présente invention, la concentration des cellules est réalisée par précipitation, co-précipitation ou précipitation par inclusion, en présence de carbonates d'ions bivalents, tels que le carbonate de calcium, le carbonate de strontium, le carbonate de baryum, et leurs mélanges.

Le milieu réactionnel est porté typiquement à une température relativement élevée, de préférence de l'ordre de 40 à 60°C, de façon à permettre la formation rapide, généralement en quelques minutes, d'un précipité contenant diverses cellules par simple décantation et sans ajout de force gravitationnelle supplémentaire. Afin d'obtenir une décantation efficace, les réactifs tels que les carbonates d'ions bivalents ne sont pas ajoutés en une seule fois dans le milieu réactionnel, mais sont ajoutés à plusieurs reprises. Après formation du précipité, sur lequel viennent s'adsorber les différentes cellules de l'échantillon, le liquide surnageant est évacué et le précipité est dissous de préférence dans un acide tel que l'acide formique ou l'acide ascorbique. Le milieu réactionnel contenant le précipité dissous est ensuite avantageusement neutralisé à l'aide d'un agent tampon.

La concentration des cellules peut également être réalisée par filtration et/ou à l'aide d'un système immuno-magnétique. Une telle concentration est alors de préférence réalisée en amont ou à la place de l'étape de concentration par précipitation, co-précipitation ou précipitation par inclusion. Une telle concentration est avantageusement envisagée pour des eaux chargées en matières en suspension et/ou en sels (eau de mer par exemple) et/ou en produits chimiques. Une telle concentration est réalisée avantageusement par l'une des deux méthodes suivantes, ou par l'association de ces deux méthodes:
- par filtration à travers une cartouche ou une membrane de taille comprise entre 10 µm et quelques millimètres, de préférence entre 10 µm et 100 ou 200 µm. L'objectif est alors de séparer les particules en suspension des cellules (micro-organismes) présentes en solution, en particulier lorsque les eaux à analyser contiennent beaucoup de matières en suspension. Cette filtration suppose des lavages qui peuvent être réalisés en mode manuel ou en automatique ;
- par une technique immuno-magnétique, avantageusement à l'aide de billes magnétiques couvertes d'un anticorps spécifique des cellules ou des micro-organismes à concentrer. Ces billes sont mises en suspension par effet magnétique dans la solution contenant les micro-organismes, après une étape de filtration préalable si nécessaire. Cette technique est particulièrement avantageuse lorsque le procédé selon la présente invention vise à détecter la présence d'organismes pathogènes dans de l'eau. Les cellules du pathogène recherché se fixent alors sur les billes par un mécanisme antigène - anticorps. Après arrêt de l'agitation magnétique, les billes sont séparées du liquide puis lavées. Une élution permet in fine de récupérer les cellules fixées dans un faible volume de liquide qui peut être analysé par la suite par le procédé de détection de la présence d'organismes pathogènes selon la présente invention, décrit ci-dessous.

Avantageusement selon la présente invention, l'adsorption des cellules sur le support solide permet de séparer les cellules viables des cellules non viables. Il apparaît en effet que les mécanismes d'adsorption mis en oeuvre pour fixer les cellules sur le support solide mettent en jeu des forces électrostatiques entre particules chargées : les particules du support solide et les cellules. Or, lorsqu'une cellule n'est plus viable, son équilibre ionique est modifié et les charges ioniques au niveau de sa membrane sont modifiées et rendent l'adsorption difficile, voire impossible. Il en résulte que le procédé selon la présente invention permet de retenir quasi essentiellement les cellules viables.

Selon une caractéristique particulière de la présente invention, le procédé d'extraction d'acides nucléiques comprend en outre une étape préalable d'ajout de calcium, avantageusement sous forme de sel.

On peut par exemple introduire de l'acétate de calcium ou tout autre sel de calcium dans le milieu réactionnel, à une teneur d'environ 10 mmol/l. Avantageusement, on procède à un ajout de calcium dans le milieu réactionnel, lorsque l'on n'a pas procédé auparavant à une concentration des cellules par précipitation, co-précipitation ou précipitation par inclusion à l'aide de carbonate de calcium.

La Demanderesse a ainsi découvert de manière surprenante que l'ajout de calcium dans le milieu réactionnel, de préférence préalablement à la lyse cellulaire et à l'adsorption des acides nucléiques sur le support solide, permettait d'aider à la fixation des acides nucléiques sur ledit support et permettait également d'activer les DNAses des cellules non viables, conduisant ainsi à la suppression de l'ADN des cellules mortes. Cette étape d'ajout de calcium est avantageusement mise en oeuvre dans le procédé selon la présente invention, puisqu'elle permet de ne conserver et de n'extraire au final que l'ADN des cellules vivantes, l'ADN des cellules mortes ayant été dénaturée. L'ajout de calcium dans le milieu réactionnel permet aussi de renforcer l'adsorption des cellules viables sur le support solide, et l'élimination des cellules non viables.

Selon une caractéristique particulière de la présente invention, le support solide contient un matériau poreux adsorbant, avantageusement un matériau vitreux ou un composé à base de silice, tel que du verre, des fibres de verre, un oxyde de silice ou un silicate. La nature du matériau est choisie de telle façon que l'on puisse réaliser une adsorption réversible des acides nucléiques sur le support solide.

Avantageusement selon la présente invention, l'adsorption des cellules sur le support est réalisée à pH neutre, avantageusement compris entre 6 et 8, encore plus avantageusement entre 7 et 7,5, encore plus avantageusement entre 7 et 7,2. Le fait d'adsorber les cellules sur le support à pH neutre permet d'obtenir un très bon rendement de fixation des cellules sur ledit support.

Le pH peut être ajusté à un pH neutre préalablement à l'adsorption des cellules, avantageusement avant de procéder à la lyse cellulaire. Ainsi, lorsque l'on procède à la concentration des cellules par précipitation, co-précipitation ou précipitation par inclusion, et que l'on aboutit à un précipité, ledit précipité est dissous de préférence dans un acide tel que l'acide formique ou l'acide ascorbique, puis le milieu réactionnel contenant le précipité dissous est ensuite avantageusement neutralisé à l'aide d'un agent tampon, tel que TRIS.

Avantageusement selon la présente invention, suite à l'adsorption des cellules sur le support solide et préalablement au séchage des cellules adsorbées sur ledit support, ledit support est lavé à l'aide d'au moins un solvant contenant des ions monovalents, avantageusement au moins un sel de sodium, tel que le thiocyanate de sodium ou le chlorure de sodium, et/ou l'EDTA. Le solvant, qui est ainsi avantageusement un agent complexant formé d'ions monovalents, permet de laver les cellules fixées sur le support solide.

On procède avantageusement à ce lavage par solvant lorsqu'au moins un sel d'ion bivalent a préalablement été introduit dans le milieu réactionnel. La demanderesse a ainsi découvert de manière surprenante que l'élution subséquente des acides nucléiques à partir du support d'adsorption pouvait être difficilement réalisée sans convertir au préalable le complexe d'acides nucléiques et d'ions bivalents, formé par exemple lors de la première étape de concentration des cellules, en complexe constitué d'acides nucléiques et d'ions monovalents. En effet, le complexe d'acides nucléiques et d'ions bivalents est fortement lié au support d'adsorption solide et ne peut pas être désorbé, puis élué par simple lavage à l'aide d'un solvant. La désorption, suivie de l'élution, des acides nucléiques n'est rendue possible que par l'ajout d'un agent complexant formé d'ions monovalents. Un tel agent permet ainsi d'échanger les ions bivalents, liés aux acides nucléiques, contre des ions monovalents et de former ainsi un nouveau complexe qui va pouvoir être désorbé beaucoup plus facilement que le complexe acides nucléiques/ions bivalents.

Selon une caractéristique particulière de la présente invention, la lyse des cellules est réalisée en effectuant un ou plusieurs cycles de chauffage, chaque cycle de chauffage consistant à chauffer les cellules adsorbées sur le support solide par micro-ondes, avantageusement à une puissance de l'ordre de 500 à 1000 Watts, encore plus avantageusement de 600 à 800 Watts, encore plus avantageusement aux alentours de 700 Watts, avantageusement pendant 10 à 60 secondes, encore plus avantageusement pendant 20 à 40 secondes, encore plus avantageusement pendant 20 à 30 secondes, puis à laisser reposer le support solide pendant 10 à 60 secondes, encore plus avantageusement pendant 10 à 40 secondes, encore plus avantageusement pendant 10 à 30 secondes. Typiquement selon la présente invention, deux à cinq, avantageusement trois à quatre, cycles de chauffage sont réalisés afin de lyser les cellules adsorbées sur le support.

Les cellules lysées restent sur le support et permettent de libérer les acides nucléiques in situ avec des rendements élevés. Les acides nucléiques ainsi libérés viennent s'adsorber sur ledit support.

Selon une caractéristique particulière de la présente invention, lors de la lyse ou suite à la lyse des cellules, on ajoute sur le support au moins un agent chaotrope, avantageusement un sel de guanidine tel que le thiocyanate de guanidine et/ou au moins un agent de saturation tel que le sperme de hareng, et/ou au moins un agent modificateur d'adsorption, avantageusement un alcool tel que l'éthanol.

L'ajout d'au moins un agent chaotrope permet de finaliser la lyse des cellules, de faciliter l'extraction d'ADN et/ou d'ARN des cellules et d'inactiver les nucléases, qui sont des enzymes susceptibles de détruire les acides nucléiques. Le sel de guanidine, tel que le thiocyanate de guanidine, est préférentiellement ajouté selon la présente invention à une concentration de plus d'une mole par litre.

L'ajout d'au moins un agent de saturation, tel que le sperme de hareng, permet de tapisser et couvrir les surfaces et parois du support solide, telles que les surfaces métalliques, sur lesquelles l'ADN pourrait s'adsorber. Cet agent de saturation permet ainsi d'assurer l'adsorption sélective des acides nucléiques sur le matériau adsorbant présent dans le support, en saturant les autres sites de fixation potentielle de l'ADN.

L'ajout d'au moins un agent modificateur d'adsorption, tel que l'éthanol, permet de laver et purifier les acides nucléiques adsorbés et d'évacuer les contaminants potentiels.

Avantageusement selon la présente invention, un mélange d'agent chaotrope et d'agent de saturation est tout d'abord ajouté au milieu réactionnel contenant les cellules. Puis, un agent modificateur d'adsorption est ajouté au milieu réactionnel dans un second temps.

Selon une caractéristique particulière de la présente invention, l'élution des acides nucléiques est réalisée par lavage du support solide à l'eau.

Le solvant pour éluer et désorber ainsi les acides nucléiques immobilisés sur le support peut être de l'eau ou tout autre solvant pouvant être compatible avec une étape ultérieure de manipulation des acides nucléiques, telle qu'une étape d'amplification desdits acides nucléiques, préférentiellement par PCR.

Avantageusement, le procédé d'extraction des acides nucléiques selon la présente invention peut être utilisé pour détecter la présence d'organismes pathogènes dans un échantillon liquide, tel que l'eau ou un liquide constitué essentiellement d'eau.

La présente invention a ainsi également pour objet un procédé de détection de la présence d'organismes pathogènes dans un échantillon liquide, caractérisé en ce qu'il comprend les étapes successives suivantes :
- une extraction d'acides nucléiques selon le procédé de la présente invention tel que mentionné ci-dessus,
- au moins une amplification d'acides nucléiques, et
- une détection qualitative et/ou quantitative d'ADN et/ou d'ARN représentatifs de la contamination du liquide par lesdits organismes pathogènes.

La présence d'agents pathogènes dans les réseaux d'alimentation en eau, tels que les réseaux d'eau potable ou d'eau industrielle, engendre de réels risques pour la santé des êtres humains. Les réseaux d'alimentation en eau contiennent ainsi pour la plupart des organismes pathogènes tels que des virus, des bactéries, des protozoaires, des champignons, des amibes ou des vers qui sont susceptibles de provoquer diverses maladies chez l'homme. Parmi ces maladies, on peut citer entre autres le choléra, la fièvre typhoïde, la diarrhée, la dysenterie, la légionellose ou encore la gastro-entérite.

Parmi les organismes pathogènes responsables de telles maladies, on peut citer plus particulièrement les protozoaires Cryptosporidium (parvum), Giardia Lamblia, les streptocoques fécaux, ainsi que les clostridium qui proviennent des rejets fécaux humains et animaux et qui sont une cause majeure de maladies gastro-entériques ; les protozoaires Toxoplasma gondii et Naegleria fowleri qui proviennent également des rejets fécaux humains et animaux et qui provoquent diverses maladies graves ; Cyclospora qui provoque des diarrhées ; les bactéries coliformes, notamment Escherichia coli, qui se trouvent dans toute la biosphère et dont la détection est importante car ils sont considérés, non pas comme des agents pathogènes typiques, mais comme des organismes indicateurs de la contamination de l'eau, puisqu'ils ne provoquent des maladies qu'à des concentrations très élevées.

Par souci de simplification dans le cadre de la présente invention, on regroupera sous le terme d'organismes pathogènes l'ensemble des organismes indicateurs de la contamination de l'eau ou du liquide analysé et des organismes pathogènes réellement responsables de maladies chez l'être humain. Peuvent également être cités parmi les organismes pathogènes responsables de maladies, les bactéries Salmonella, dont les sérotypes S. enteridis, S. enterica, S. thyphi et S. paratyphi, qui sont à l'origine de 70 % des maladies bactériennes d'origine alimentaire, notamment des fièvres, typhoïdes et paratyphoïdes, et qui sont à l'origine d'un grand nombre de décès ; Helicobacter pylori qui est un organisme lié à l'origine d'au moins 75 % de l'ensemble des ulcères de l'estomac et à deux types de cancers de l'estomac, et qui est également à l'origine d'un grand nombre de décès; Legionella, que l'on trouve dans les zones naturelles ainsi que dans les systèmes de chauffage de l'eau, qui provoque la maladie du légionnaire ; ainsi que les virus d'origine humaine et animale, et notamment les virus de l'hépatite A, les virus de type Norwalk, les rotavirus, des adénovirus, les entérovirus et les rhéovirus. Les virus Norwalk provoquent notamment des maladies gastro-intestinales sporadiques et épidémiques avec diarrhée. On en voit apparaître environ 200 000 cas par an aux Etats-Unis.

Etant donné la présence de divers agents pathogènes dans l'ensemble des réseaux d'alimentation en eau, ces derniers doivent être contrôlés et éventuellement désinfectés, avant de pouvoir considérer l'eau issue de ces réseaux comme potable. En ce qui concerne les réseaux d'alimentation en eau usée ou en eau de refroidissement ainsi que les réseaux de production d'énergie, notamment ceux de production d'énergie nucléaire, il est parfois nécessaire d'analyser l'eau et même de la désinfecter avant de pouvoir la réutiliser ou la rejeter dans le milieu environnant.

La détection de la présence d'organismes pathogènes, notamment la détection des organismes hautement pathogènes à croissance lente, qui sont les plus difficiles à détecter, représente ainsi un enjeu majeur dans les milieux du traitement de l'eau, de la distribution de l'eau ainsi que du contrôle de la qualité de l'eau. Les techniques traditionnelles de détection d'agents pathogènes développées jusqu'à présent présentent divers inconvénients : elles ne sont pas exploitées en continu ou en temps réel, elles sont de sensibilité peu élevée vis-à-vis des organismes pathogènes et elles s'avèrent être longues et coûteuses en termes de matériel et de personnel.

Le procédé de détection de la présence d'organismes pathogènes dans un échantillon selon la présente invention permet ainsi d'effectuer une détection de plusieurs micro-organismes pathogènes, avantageusement en ligne en un temps court, à moindre coût et avec une sensibilité et une spécificité élevées, permettant de parvenir à un niveau de détection d'un organisme dans 100 ml d'échantillon de liquide analysé.

Avantageusement selon la présente invention, les organismes pathogènes détectés sont des bactéries, des virus, des champignons et/ou des protozoaires.

Avantageusement selon la présente invention, le volume initial d'échantillon est inférieur à 5 litres, avantageusement inférieur à 2 litres, encore plus avantageusement inférieur à 1 litre, encore plus avantageusement entre 50 ml et 1 litre, encore plus avantageusement entre 100 ml et 1 litre.

Lorsque le volume initial d'échantillon est de l'ordre de 1 à 2 litres, les techniques directes de lyse des cellules des organismes ne sont pas réalisables sur des volumes aussi importants, et les organismes pathogènes, que l'on cherche à détecter selon le procédé de la présente invention, doivent être concentrés à des volumes inférieurs à 100 ml avant de pouvoir démarrer la suite du traitement. En outre, les normes de qualité d'eau potable imposent de prouver l'absence d'un organisme dans 100 ml d'échantillon d'eau analysée. La difficulté de la concentration d'organismes pathogènes issus d'échantillons de l'ordre du litre est de perdre le moins possible d'organismes, tout en réduisant le volume de l'échantillon jusqu'à un volume de l'ordre d'une centaine de ml.

Avantageusement selon la présente invention, la concentration des organismes pathogènes est réalisée par précipitation, co-précipitation ou précipitation par inclusion. Une telle concentration d'organismes à partir de volumes d'échantillons de l'ordre du litre permet ainsi d'avoir un procédé de haute sensibilité et de respecter les normes imposées sur la qualité de l'eau. En effet, plus un volume d'échantillon est important, plus la sensibilité est accrue et plus les données d'analyse sont précises. En outre, le procédé de détection selon la présente invention permet dé détecter la présence de moins de 10 espèces différentes dans des volumes initiaux d'échantillons de 100 ml à 2 litres, de préférence de 1 à 2 litres.

L'étape de lyse cellulaire selon la présente invention est effectuée en utilisant de préférence des volumes d'échantillons analysés de l'ordre de 10 à 200 ml, de préférence de 40 à 50 ml.

Selon la présente invention, une amplification d'acides nucléiques est réalisée dans le procédé de détection suite à l'extraction desdits acides nucléiques, qui est avantageusement réalisée par adsorption réversible des acides nucléiques sur un support solide contenant un matériau adsorbant, suivie d'une élution de ces acides. Cette amplification est effectuée afin de pouvoir détecter au minimum un agent pathogène, et de préférence de 1 à 5 agents pathogènes, dans l'échantillon de liquide analysé.

Avantageusement selon la présente invention, l'amplification d'acides nucléiques est réalisée par au moins une réaction en chaîne par polymérase (PCR) avantageusement en temps réel. La PCR est une technique d'amplification communément utilisée. La technique de PCR (Rolfs *et al.* 1991) nécessite le choix de paires d'amorces oligonucléotidiques encadrant le fragment qui doit être amplifié. Les principes généraux et les conditions de réalisation de l'amplification d'acides nucléiques par PCR sont bien connus de l'homme du métier et sont notamment décrits dans les brevets US 4 683 202, US 4 683 195 et US 4 965 188.

D'autres techniques similaires d'amplification d'acides nucléiques (ADN et/ou ARN) peuvent être avantageusement employées comme alternative à la PCR (PCR-like) à l'aide de couple d'amorces de séquences nucléotidiques. Par PCR-like, on entend désigner toutes les méthodes mettant en oeuvre des reproductions directes ou indirectes des séquences d'acides nucléiques, ou bien dans lesquelles les systèmes de marquage ont été amplifiés, ces techniques étant bien entendu connues de l'homme du métier. On peut notamment citer la technique SDA (Strand Displacement Amplification) ou technique d'amplification à déplacement de brin (Walker *et al.,* 1992), la technique TAS (Transcription-based Amplification System) décrite par Kwoh *et al.* (1989), la technique 3SR (Self-Sustained Sequence Replication) décrite par Guatelli et al. (1990), la technique NASBA (Nucleic Acid Sequence Based Amplification) décrite par Kievitis et al. (1991), la technique TMA (Transcription Mediated Amplification), la technique LCR (Ligase Chain Reaction) décrite par Landegren *et al.* (1988), la technique de RCR (Repair Chain Reaction) décrite par Segev (1992), la technique CPR (Cycling Probe Reaction) décrite par Duck *et al.* (1990), la technique d'amplification à la Q-béta-réplicase décrite par Miele *et al.* (1983). Certaines de ces techniques ont depuis été perfectionnées. Ainsi, selon la présente invention, une détection spécifique d'agents pathogènes peut être effectuée en utilisant de telles techniques d'amplification d'acides nucléiques à grande vitesse.

En comparant la technique d'amplification par PCR ou par une technique similaire telle que la NASBA avec des techniques traditionnelles pour la détection d'agents pathogènes telles que la culture cellulaire, la Demanderesse a découvert que l'on pouvait réduire la durée requise pour l'essai de quelques semaines ou quelques jours à quelques heures. En outre, l'amplification par PCR ou analogue est facile à réaliser et les coûts initiaux comme les coûts ultérieurs pour la mise en oeuvre de la PCR s'avèrent bien inférieurs aux coûts engendrés pour la mise en oeuvre des techniques de cultures cellulaires. De plus, on peut utiliser une PCR pour identifier une forme spécifique des agents pathogènes que l'on trouve dans les liquides tels que l'eau. On peut ainsi l'utiliser pour détecter l'état infectieux d'un organisme, prouvant la présence ou l'absence d'ADN ou d'ARN spécifique de l'agent pathogène. L'amplification par PCR selon l'invention permet aussi d'obtenir un procédé de sensibilité élevée, avantageusement inférieure à 1 picogramme, et de manière encore plus avantageuse inférieure à quelques femtogrammes d'acides nucléiques.

La Demanderesse a également découvert que l'utilisation de l'amplification par PCR (ou analogue) suivie d'une détection spécifique in situ, en temps réel, par exemple par fluorescence fournissait des données d'analyse relatives à la détection et à l'identification des organismes plus rapidement et plus précisément que tout autre système de détection à base de biopuces. Les biopuces permettent de détecter un nombre d'espèces pathogènes beaucoup plus important que celui détecté à l'aide de la PCR en ligne mais, sur la base d'agents pathogènes connus (qui peuvent être plus efficacement testés en tant que groupes), la PCR en temps réel est beaucoup plus efficace et donne une bien meilleure spécificité que dés biopuces standard hybridées. En outre, une hybridation de puces, qui permet une mise en contact des micro-organismes avec une sonde à ADN et/ou à ARN, prend plusieurs heures, jusqu'à inclure souvent une incubation pendant la nuit, ce qui est significativement plus long que l'amplification de l'ADN ou de l'ARN en temps réel.

Pour effectuer l'amplification d'acides nucléiques, de préférence par PCR, selon la présente invention, une grande variété d'amorces peut être utilisée, certaines d'entre elles étant déjà disponibles dans des bases de données ou dans la littérature, certaines autres ayant déjà été mises au point pour des organismes spécifiques.

Selon un mode de réalisation particulier de la présente invention, on utilise une "nested" PCR, en divisant la PCR en deux étapes, afin d'obtenir une sensibilité maximale vis-à-vis des agents pathogènes que l'on cherche à détecter. La première PCR est alors faite avec la totalité de l'échantillon (typiquement de plusieurs centaines de micro litres avec seulement quelques molécules d'ADN) en utilisant un ensemble d'amorces dégénérées employant un appareil à cycles thermiques standard. Après amplification pendant quelques cycles (par exemple de 10 à 20 cycles) à haute température (75 à 100°C), le fluide qui contient alors au moins plusieurs milliers d'exemplaires de chaque ADN peut être divisé en un certain nombre de petits flacons dans un second appareil de PCR, en utilisant un moyen de prélèvement et de distribution automatiques tel qu'un robot. Un second ensemble d'amorces est ensuite ajouté à chaque flacon et une seconde amplification d'acides nucléiques est alors réalisée tout en effectuant une détection en ligne, par exemple par fluorescence. La première amplification va ainsi pouvoir être utilisée pour amplifier les acides nucléiques de groupes d'agents pathogènes à l'aide d'amorces universelles qui sont dégénérées, alors que la seconde amplification va permettre de déterminer précisément les espèces présentes dans l'échantilion analysé à l'aide d'amorces spécifiques de chaque espèce que l'on cherche à détecter. Dans ce mode de réalisation particulier de la présente invention, la détection est effectuée lors de la réalisation de la deuxième PCR ; aucune détection n'est effectuée lors de la première PCR qui se limite à un rôle d'amplification des acides nucléiques.

Avantageusement selon la présente invention, le procédé peut contenir en outre une étape de concentration de volume, afin de parvenir à des volumes de l'ordre d'une centaine de µl jusqu'à moins de 1 ml, avantageusement moins de 100 µl, avant de procéder à l'amplification des acides nucléiques et suite à l'étape d'extraction/concentration des acides nucléiques.

Avantageusement selon la présente invention, l'étape de détection des organismes pathogènes, qui suit celle d'amplification des acides nucléiques, est une détection en ligne (en temps réel), in situ. De manière encore plus avantageuse, cette détection est réalisée par fluorescence, par luminescence ou par spectrométrie de masse. Elle peut également être réalisée par d'autres techniques de détection bien connues de l'homme du métier, telles que celles d'hybridation sur puce ou d'amplification enzymatique.

De manière encore plus avantageuse selon l'invention, la détection est réalisée in situ par fluorescence. Afin d'obtenir un signal de détection, on peut ainsi ajouter lors de l'étape d'amplification du Cybergreen ou un autre fluorophore d'intercalation à deux brins permettant d'indiquer le succès de l'amplification par une forte augmentation de fluorescence au cours du cycle. En effet, les amorces marquées à l'aide des fluorophores s'hybrident spécifiquement avec l'ADN cible de l'échantillon. La réaction d'amplification par PCR engendrant un grand nombre de copies de l'ADN cible, on peut ainsi obtenir une quantification de l'intensité du signal de fluorescence, directement corrélé avec la quantité d'ADN cible initiale dans l'échantillon analysé.

Pour obtenir une détection multispécifique, c'est-à-dire une détection spécifique de plusieurs agents pathogènes, dans un seul flacon et afin d'augmenter la spécificité vis-à-vis de ces agents pathogènes si cela est nécessaire, des fanaux moléculaires ou des traceurs similaires peuvent être ajoutés aux différents flacons utilisés lors de l'amplification. En utilisant une instrumentation standard, on peut détecter jusqu'à quatre espèces différentes par flacon.

Avantageusement, le procédé de détection selon la présente invention peut contenir en outre une étape préalable de traitement de l'échantillon liquide par choc thermique et/ou par ajout d'un composé chimique inducteur de gène afin d'induire l'ARN de gènes spécifiques et de détecter les organismes pathogènes viables dans ledit échantillon liquide. En effet, un choc thermique (augmentation de la température pendant quelques instants) ou l'ajout d'un composé chimique induisent un ensemble de protéines permettant la réalisation d'une réaction RT (de transcriptase inverse) en tant que première étape, lorsqu'est réalisée la première PCR, permettant ainsi d'obtenir un signal spécifique d'organismes viables. La réaction RT permet de transformer l'ARN en ADN. Si cela s'avère nécessaire, des ADNases peuvent être ajoutées pour détruire l'ADN natif Une spécificité est obtenue en induisant un ADN spécifique de haut niveau.

Avantageusement selon la présente invention, un choc thermique est réalisé par impulsion de chaleur durant moins de 3 heures, avantageusement à une température de 30 à 50°C.

Avantageusement selon la présente invention, un composé chimique inducteur de gène est ajouté. Un tel composé permet d'induire l'ARN de gènes spécifiques. Le composé est avantageusement un élément nutritif tel que le lactose ou un inducteur ciblé comme l'IPTG (Isopropyl-(beta)-D-thiogalactopyranoside). Ce composé chimique permet une forte augmentation du niveau des ARN spécifiques dans les cellules. La durée de l'induction dépend de l'organisme, mais est typiquement comprise entre 2 minutes et 1 heure. Les protéines induites par la chaleur comme dnaJ, grpE, hscB, hslJ, hslV, htpG, htpX, htrB, htrC, pphA, pphB, rpoE et d'autres protéines de choc (par exemple des classes des petites HSP, HSP 60, HSP 70, HSP 90, HSP 100, ubiquitine) sont des cibles efficaces.

Dans le cadre de la présente invention, l'induction d'ARN de gènes spécifiques peut également être réalisée par une irradiation aux UV, par l'utilisation de substances toxiques telles que le cis-platinum, l'anisomycine, la tunicamycine, l'arsénite ou par un choc osmotique.

L'induction est une preuve directe de la viabilité de la cellule. De plus, un ARN peut ainsi être détecté à de nombreux exemplaires avec une plus grande sensibilité par rapport à l'ADN. Alors que la détection d'ADN est importante, puisque l'ADN est présent dans chaque cellule en une quantité fixe, et qu'il est alors aisé de faire une corrélation entre l'ADN et le nombre de cellules, l'ARN quant à lui peut être induit à différents niveaux, permettant ainsi de détecter la viabilité des organismes pathogènes.

Avantageusement selon la présente invention, le composé chimique inducteur de gène est le lactose ou l'IPTG afin d'induire l'ARN du lac Z. L'opéron lac Z, qui est unique dans sa séquence dans un certain nombre d'organismes pathogènes, constitue en effet une cible utile. La durée de l'induction est de quelques minutes.

Avantageusement selon la présente invention, la détection des organismes pathogènes viables dans un échantillon liquide est réalisée après avoir obtenu un premier signal de détection d'ADN représentatif de la présence d'organismes pathogènes dans ledit échantillon. Ainsi, dans un mode de réalisation particulier de la présente invention, l'ensemble des étapes du procédé de détection de la présence d'organismes pathogènes sont tout d'abord mises en oeuvre afin de conclure sur la contamination effective (ou non) de l'échantillon de liquide analysé. En cas de réponse positive, par l'obtention d'un signal d'ADN représentatif de la présence d'organismes pathogènes dans ledit échantillon, l'ensemble des étapes du procédé de détection selon la présente invention sont à nouveau mises en oeuvre, en ajoutant une étape préalable de traitement de l'échantillon par choc thermique et/ou par ajout d'un composé chimique inducteur de gène afin d'induire l'ARN de gènes spécifiques. Le signal d'ADN est ensuite comparé avec le signal d'ARN obtenu, ce qui permet de détecter les organismes pathogènes viables dans échantillon analysé.

Dans un mode de réalisation particulier de la présente invention, l'ensemble des étapes séquentielles du procédé sont gérées de manière entièrement automatique à l'aide d'une logique de commande, telle qu'un ordinateur.

Avantageusement selon la présente invention, la logique de commande est constituée par au moins un ordinateur, par exemple de type PC, qui commande l'ensemble des étapes. La logique de commande permet ainsi de déclencher et de contrôler les différentes étapes du procédé et de recueillir, à l'issue du procédé, les données d'analyse relatives à la détection des organismes.

Avantageusement selon la présente invention, les données d'analyse relatives à la détection des organismes sont transférées de manière automatique à un système de commande central. Le système de commande central selon la présente invention est avantageusement constitué par au moins un ordinateur. Ce système de commande central va pouvoir regrouper, analyser et gérer l'ensemble des données analytiques, par exemple à l'aide de bases de données, et incorporer ces données dans des modèles mathématiques qui vont permettre de prédire le taux de contamination des liquides analysés, le type de micro-organismes présents dans ces liquides ainsi que le taux de survie de ces micro-organismes.

Avantageusement selon la présente invention, le procédé fonctionne en continu, de manière autonome, pendant une durée supérieure ou égale à un jour, de préférence de trois jours à sept jours. La durée de fonctionnement du procédé peut varier en fonction notamment du nombre de réactifs utilisés.

En outre, un échantillon de liquide donné peut être dosé et analysé plusieurs fois par jour en utilisant le procédé de détection selon la présente invention. Pour ce faire, des séquences d'ADN (amorces) spécifiques sont utilisées, et si cela est nécessaire de nouvelles amorces peuvent être mises au point. La durée du dosage pour un cycle de test en utilisant le procédé selon la présente invention est optimisée à une durée de l'ordre du temps de doublement de la plupart des organismes (4 à 6 heures).

La présente invention a également pour objet un dispositif d'extraction de composés ou complexes intracellulaires, tels que des acides nucléiques, à partir d'un échantillon contenant des cellules en suspension dans un liquide, caractérisé en ce qu'il comprend :
- au moins un moyen de fixation des cellules,
- au moins un moyen de séchage des cellules fixées,
- au moins un moyen de lyse des cellules par micro-ondes, et
- au moins un moyen de récupération desdits composés ou complexes intracellulaires.

Ce dispositif convient à la mise en oeuvre du procédé d'extraction des composés ou complexes intracellulaires selon la présente invention. Les composés ou complexes intracellulaires peuvent être tout composé biologique tel que des protéines, notamment des protéines recombinantes, non sécrétées, ou des acides nucléiques, ou encore des organites.

Avantageusement selon la présente invention, le liquide est de l'eau ou un liquide constitué essentiellement d'eau.

Avantageusement selon la présente invention, le moyen de fixation des cellules comprend un ou plusieurs support(s) solide(s) contenant au moins un matériau poreux, avantageusement un matériau d'adsorption réversible, tel qu'un matériau vitreux ou un composé à base de silice, tel que du verre, des fibres de verre, un oxyde de silice ou un silicate.

Dans un mode de réalisation particulier de la présente invention, le moyen de séchage des cellules fixées comprend au moins un système d'injection d'air, avantageusement d'air comprimé, ou un système de séchage sous vide. Un système d'injection de tout autre gaz, tel que l'azote, peut également être utilisé pour sécher les cellules fixées sur le support solide, et éliminer ainsi le liquide extracellulaire. A titre de système d'injection d'air, on peut utiliser un compresseur fournissant une pression entre 1 et 2 bars.

Avantageusement selon la présente invention, le moyen de lyse des cellules contient au moins un magnétron, tel que ceux que l'on trouve dans les fours à micro-ondes commerciaux.

Avantageusement selon la présente invention, le moyen de récupération des composés ou complexes intracellulaires comprend un moyen de fixation desdits composés ou complexes intracellulaires, ledit moyen de fixation contenant un ou plusieurs support(s) solide(s) contenant au moins un matériau poreux. Le matériau poreux est avantageusement un matériau d'adsorption réversible, tel qu'un matériau vitreux ou un composé à base de silice, tel que du verre, des fibres de verre, un oxyde de silice ou un silicate.

De manière encore plus avantageuse selon la présente invention, le moyen de fixation des cellules et le moyen de fixation des composés ou complexes intracellulaires sont confondus. Un tel mode de réalisation est particulièrement intéressant, puisqu'il permet de n'utiliser qu'un seul support solide pour fixer les cellules, ainsi que les composés ou complexes intracellulaires, tels que les acides nucléiques, extraits des cellules lysées.

La fixation des cellules et des acides nucléiques peut ainsi être réalisée par exemple à l'aide d'une colonne d'absorption poreuse contenant un matériau vitreux, avantageusement comprenant des groupes hydroxy en surface, ou un matériau à base de silice, qui va permettre l'adsorption des cellules et des acides nucléiques sur le support solide.

De manière encore plus avantageuse selon la présente invention, le(s) support(s) solide(s) pour fixer les cellules et/ou les composés ou complexes intracellulaires est (sont) constitué(s) par des plaques de micro-titration contenant des multi-puits, avantageusement des plaques de 60, 96 ou 384 puits, chaque puit contenant au moins un matériau poreux, avantageusement adsorbant, tel qu'un matériau vitreux ou un composé à base de silice, par exemple du verre, des fibres de verre, un oxyde de silice ou un silicate. Chaque puits correspond alors à une micro-colonne d'adsorption réversible.

De manière encore plus avantageuse selon la présente invention, les puits des plaques de micro-titration ne sont utilisés qu'une seule fois et sont déplacés automatiquement pour recevoir l'échantillon liquide. De l'air sous pression peut être injecté pour entraîner le fluide dans les puits. La plaque est référencée par rapport à un système de coordonnées XY, X correspondant à l'axe suivant la largeur de la plaque et Y correspondant à l'axe suivant la longueur de la plaque. Le déplacement automatique de la plaque, et en conséquence celui des puits qui sont fixés sur la plaque, est effectué selon les axes X et Y, par exemple à l'aide du bras d'un robot. Le déplacement automatique peut être actionné par la logique de commande selon la présente invention. Dans un mode de réalisation particulier de la présente invention, le déplacement automatique est réalisé à l'aide d'un robot, qui est commandé à l'aide d'une logique de commande, avantageusement constituée par un ou plusieurs ordinateur(s). Avantageusement, le robot a un bras qui peut se déplacer dans l'espace, selon un référentiel XYZ.

Avantageusement selon la présente invention, les puits des plaques micro-titration peuvent être protégés contre l'évaporation par une goutte de fluide ayant une faible densité spécifique ou par un couvercle transparent.

Dans un mode de réalisation particulier de la présente invention, le dispositif d'extraction contient en outre un moyen de concentration des cellules par précipitation, co-précipitation ou précipitation par inclusion.

Avantageusement selon la présente invention, le moyen de concentration des cellules par précipitation, co-précipitation ou précipitation par inclusion est un réacteur comprenant un système d'introduction de l'échantillon liquide, avantageusement constitué de valves, un système d'introduction de différents réactifs, avantageusement constitué de tubes, de valves et de pompes rotatives ou à galet, un système d'agitation, un système d'évacuation du liquide surnageant, avantageusement constitué de tubes et de valves, un système d'évacuation du précipité, avantageusement constitué de valves et de pompes, et avantageusement placé au fond du réacteur, et un système de chauffage, avantageusement constitué d'une enveloppe (gaine) chauffante placée autour du réacteur.

Le réacteur selon la présente invention est de préférence en métal ou en plastique, avantageusement en acier inoxydable ou en polyéthylène, avantageusement en acier inoxydable, afin d'obtenir une résistance élevée aux réactifs chimiques introduits dans ledit réacteur. Le réacteur occupe avantageusement un volume de 100 ml à 10 litres, de préférence de 1 à 3 litres. Les tubes sont quant à eux avantageusement en acier ou en plastique.

L'introduction de l'échantillon selon la présente invention peut être réalisée de manière automatique par l'intermédiaire de valves qui sont ouvertes et refermées à l'aide de la logique de commande. Selon la présente invention, l'introduction des différents réactifs nécessaires à la concentration des cellules est effectuée à l'aide de tubes, de valves et de pompes rotatives ou à galet, lesdites valves et pompes étant actionnées également par la logique de commande. L'agitation et le chauffage sont également commandés par la logique de commande. La température interne du réacteur est avantageusement contrôlée à l'aide de capteurs.

La présente invention a également pour objet un dispositif de détection de la présence d'organismes pathogènes dans un échantillon liquide, tel que l'eau, comprenant :
- un dispositif d'extraction d'acides nucléiques selon la présente invention tel que mentionné ci-dessus,
- un moyen d'amplification d'acides nucléiques,
- un moyen de détection qualitative et/ou quantitative d'ADN et/ou d'ARN représentatifs de la contamination du liquide par lesdits organismes pathogènes,
- des moyens de pompage, et
- des moyens de prélèvement et de distribution avantageusement automatiques, tels qu'un robot.

Le dispositif de détection selon la présente invention convient à la mise en oeuvre du procédé de détection d'organismes pathogènes selon la présente invention.

Avantageusement selon la présente invention, à l'issue de l'extraction d'acides nucléiques et après élution des différents échantillons d'acides nucléiques présents dans les puits utilisés lors de l'adsorption, tous les échantillons liquides sont rassemblés afin de pouvoir procéder aux étapes suivantes d'amplification des acides nucléiques et de détection des organismes pathogènes.

Avantageusement selon la présente invention, le moyen d'amplification des acides nucléiques contient au moins un appareil de réaction en chaîne par polymérase à thermocycle. L'appareil PCR à thermocycle selon la présente invention réalise des cycles d'amplification d'acides nucléiques successifs, chaque cycle étant réalisé à une plage de température et pendant une durée données. De manière encore plus avantageuse selon la présente invention, le moyen d'amplification est constitué par deux appareils de réaction en chaîne par polymérase à thermocycle afin d'obtenir une sensibilité maximale vis-à-vis des agents pathogènes que l'on cherche à détecter dans l'eau, notamment lorsque l'on procède à une "nested" PCR. Les deux appareils sont alors utilisés séquentiellement. Le passage des acides nucléiques d'un appareil à l'autre est réalisé à l'aide de moyens de prélèvement et de distribution automatiques et avantageusement d'une logique de commande qui commande ces moyens.

Dans un mode de réalisation particulier de la présente invention, un appareil à thermocycle avec détection en ligne intégré est utilisé pour détecter l'amplification de l'ADN spécifique. Ce type d'appareil est ainsi constitué d'un appareil PCR à thermocycle avec chambre à détection en ligne intégrée. Dans le cas particulier selon la présente invention de la réalisation d'une amplification par "nested" PCR, la deuxième PCR est réalisée dans ce type d'appareil, la première PCR étant réalisée dans un appareil à thermocycle classique. Un tel appareil peut être acquis auprès de certaines sociétés telles que la société Roche Molecular Biochemicals. Dans ce mode de réalisation particulier de la présente invention, les moyens d'amplification et de détection sont rassemblés dans un même appareil.

Avantageusement selon la présente invention, le moyen de détection contient au moins un détecteur à fluorescence. Le détecteur à fluorescence comprend avantageusement un module optique tel qu'une caméra électronique qui enregistre les photons issus des fluorophores et qui les convertit en électrons. L'image électronique formée est ensuite avantageusement transférée vers la logique de commande.

Avantageusement selon la présente invention, l'échantillon contenant les organismes pathogènes circule au travers du dispositif d'extraction d'acides nucléiques selon la présente invention à l'aide de moyens de pompage, avantageusement constitués par des tubes équipés de valves et/ou de pompes. Les moyens de pompage constituent ainsi un système fluidique. Les moyens de pompage selon l'invention sont de préférence actionnés et contrôlés par une logique de commande.

Avantageusement selon la présente invention, la manipulation des acides nucléiques, après réalisation de la concentration/extraction d'ADN et/ou d'ARN, est effectuée à partir de l'entrée du moyen d'amplification des acides nucléiques jusqu'à la sortie du moyen de détection des acides nucléiques, à l'aide de moyens de prélèvement et de distribution avantageusement automatiques, de préférence constitués par un robot. Ainsi, les moyens d'amplification et de détection des acides nucléiques ne comportent pas de connexion directe entre elles (pas de système fluidique), contrairement au dispositif d'extraction d'acides nucléiques selon la présente invention. Les moyens de prélèvement et de distribution avantageusement automatiques selon l'invention sont actionnés et contrôlés de préférence par la logique de commande.

Le dispositif de détection selon la présente invention contient ainsi avantageusement en outre une logique de commande. La logique de commande contient de préférence au moins un ordinateur, par exemple de type PC, qui commande l'ensemble du dispositif et qui reçoit, gère et traite l'ensemble des données d'analyse relatives à la détection des organismes pathogènes.

Avantageusement, la logique de commande selon la présente invention est en interface avec un réseau pour transférer, de manière automatique ou sur requête, les données d'analyse relatives à la détection des organismes à un système de commande central.

Avantageusement selon la présente invention, le système de commande central est constitué par au moins un ordinateur. Le système de commande central peut ainsi recevoir et enregistrer les données acquises, qui sont ensuite analysées, contrôlées et gérées.

Avantageusement selon la présente invention, le dispositif contient en outre une zone de refroidissement pour conserver les réactifs sensibles, de préférence un bloc de refroidissement, qui est protégé contre la condensation de la vapeur d'eau.

Avantageusement selon la présente invention, le dispositif est directement relié au réseau de distribution d'eau potable pour échantillonnage automatique. Le dispositif de détection selon la présente invention permet ainsi de tester de façon entièrement automatisée des échantillons prélevés dans différents réseaux d'eau, notamment celui d'eau potable. Il peut également être réutilisé de nombreuses fois, sans avoir besoin de procéder au changement des différents moyens utilisés.

Typiquement, la succession des différentes étapes du procédé de détection d'organismes pathogènes selon la présente invention peut être avantageusement mise en oeuvre comme suit :
1. Le moyen de concentration des cellules par précipitation, co-précipitation ou précipitation par inclusion est purgé, nettoyé puis rempli d'un volume de 100 ml à 2 litres de liquide à analyser, de préférence de 1 à 2 litres d'eau. L'introduction du liquide dans le réacteur est effectuée à l'aide de valves, dont l'ouverture est actionnée par un ordinateur.
2. Divers réactifs, tels que du carbonate de sodium et de l'acétate de calcium, sont ajoutés dans le réacteur par l'intermédiaire de valves et de pompes rotatives, qui sont commandées par l'ordinateur. Un précipité d'inclusion est alors formé au fond de la chambre, sur lequel viennent s'adsorber les cellules et les acides nucléiques des organismes pathogènes.
3. Le liquide surnageant est évacué, par exemple par gravité, à l'aide d'un tube et d'une valve.
4. Le précipité est dissous à l'aide d'un acide, ajouté dans le réacteur par l'intermédiaire d'une pompe, qui est actionnée par l'ordinateur. Un agent tampon est ajouté également de la même manière afin d'ajuster le pH à un pH neutre, compris par exemple entre 7 et 7,5. A ce stade, le volume de solution est alors de 10 à 100 ml, de préférence de 40 à 50 ml.
5. La solution contenant les cellules (organismes pathogènes) est transférée sur un support solide, avantageusement sur des plaques de micro-titration contenant des puits, lesdits puits contenant eux mêmes un matériau adsorbant, tel qu'un matériau vitreux. De l'air sous pression peut être injecté pour entraîner le fluide dans les puits et un référentiel XY ou XYZ est utilisé pour manipuler la matrice de colonnes adsorbantes. La manipulation des puits, qui sont chacun des micro-colonnes, peut ainsi être effectuée à l'aide d'un robot actionné par l'ordinateur, dont le bras vient déplacer la plaque où se trouvent les puits selon un référentiel XY ou XYZ.
6. Les cellules sont adsorbées sur ledit support solide qui constitue un moyen d'adsorption et de concentration des cellules.
7. Le support est ensuite lavé à l'aide de chlorure de sodium et d'EDTA par ajout par l'intermédiaire de valves et de pompes, commandées par l'ordinateur, afin de remplacer les ions bivalents complexés aux acides nucléiques par des ions monovalents. Le complexe acides nucléiques/ions monovalents ainsi formé est alors à ce stade toujours lié aux micro-colonnes d'adsorption.
8. La lyse des cellules adsorbées est alors réalisée en séchant tout d'abord le support par injection d'air afin d'éliminer le liquide extracellulaire, puis en introduisant le support, avantageusement les plaques de micro-titration contenant des puits contenant au moins un matériau adsorbant, dans un four à micro-ondes. Quatre cycles de chauffage sont réalisés avec une fréquence de radiation de l'ordre 2,5 GHz, chaque cycle de chauffage consistant en un chauffage des cellules par micro-ondes à une puissance de l'ordre de 700 Watts pendant 20 à 30 secondes, puis en laissant reposer et refroidir le support pendant 10 à 30 secondes.
9. Les cellules lysées restent sur le support et permettent de libérer les acides nucléiques in situ avec des rendements élevés. L'ADN et/ou l'ARN libéré des cellules vient alors s'adsorber naturellement sur le support solide, avantageusement sur des plaques de micro-titration contenant des puits, lesdits puits contenant eux mêmes un matériau adsorbant, tel qu'un matériau vitreux.
10. Un agent chaotrope, tel que le thiocyanate de guanidine, et un agent de saturation, tel que du sperme de hareng sont alors ajoutés par l'intermédiaire de valves et de pompes, commandées par l'ordinateur. Un agent modificateur d'adsorption, tel que l'éthanol, est ensuite ajouté par l'intermédiaire de valves et de pompes, commandées par l'ordinateur.
11. Les acides nucléiques sont ensuite élués par ajout d'un tampon, tel que l'eau, à l'aide d'un robot.
12. Les acides nucléiques sont rassemblés dans un tube manipulé par exemple par un robot. A partir de ce stade, toutes les manipulations sont faites à l'aide de moyens de prélèvement et de distribution automatiques tels qu'un robot.
13. Eventuellement, et si cela s'avère nécessaire, le volume total de solution contenant les différents acides nucléiques rassemblés et élués est diminué pour parvenir à des volumes de l'ordre d'une centaine de µl jusqu'à moins de 1 ml, avant de procéder à l'amplification des acides.
14. Les acides nucléiques sont transférés dans un appareil à thermocycle standard, à l'aide du robot et la première amplification est alors réalisée dans l'appareil en ajoutant tout d'abord les réactifs pour procéder à la PCR tels que le tampon de PCR, l'ensemble d'amorces 1, le TAQ, les nucléotides.
15. Le mélange d'acides amplifiés une première fois est ensuite séparé dans plusieurs flacons afin d'obtenir une sensibilité maximale vis-à-vis des agents pathogènes que l'on cherche à détecter.
16. Une seconde amplification est alors réalisée dans un second appareil à thermocycle en temps réel, en ajoutant notamment l'ensemble d'amorces 2 (TAQ, nucléotides). Des agents de visualisation et de détection, tels que des agents de fluorescence, sont également ajoutés. Les agents de détection vont ainsi pouvoir venir s'hybrider sur les fragments des germes amplifiés. Toutes les manipulations d'échantillons (transferts, ajouts des différents réactifs,...) sont manipulés à l'aide du robot mentionné ci-dessus.
17. La fluorescence est contrôlée et détectée in situ lors de la seconde amplification (détection en ligne).
18. Les données d'analyse relatives à la détection des organismes pathogènes sont visualisées sur l'ordinateur et transférées à un système de commande central. L'ensemble des étapes séquentielles du procédé sont gérées et contrôlées par une logique de commande constituée par un ordinateur.

Les exemples suivants sont donnés à titre non limitatif et illustrent la présente invention.

### Exemples de réalisation de l'invention :

### Exemple 1: Précipitation par inclusions d'agents pathogènes

On recueille tout d'abord un échantillon d'eau, prélevé directement dans le réseau d'alimentation en eau potable, dans l'environnement ou provenant d'un laboratoire ou d'une autre source, dans un réacteur de 2 litres et on le chauffe à 43°C.
10 ml de TRIS 1 M (agent tampon) sont d'abord ajoutés pour parvenir à un pH de 8,5 et 25 ml d'acétate de calcium 1M sont ensuite ajoutés. Les deux composés sont alors mélangés rapidement, puis l'opération de mélange est stoppée.
10 ml de Na₂CO₃ 1 M sont ensuite ajoutés et on fait réagir les différents composés pendant 3 minutes. On mélange avec précaution pendant environ 5 secondes, et on ajoute alors 10 ml de Na₂CO₃ puis on fait réagir le milieu réactionnel pendant encore 3 minutes. On mélange doucement mais suffisamment et on fait à nouveau réagir le milieu réactionnel pendant encore 10 minutes. On ajoute 7,5 ml d'acétate de calcium 1M et on fait réagir pendant 3 minutes. On mélange avec précaution, on ajoute 10 ml de Na₂CO₃ et on fait encore réagir pendant 5 minutes.
On décante le surnageant en ouvrant une vanne. On dissout le précipité en ajoutant 60 ml d'acide ascorbique à 20 %. On tamponne ensuite le mélange en ajoutant de la base TRIS 2 M pour élever le pH à 7,2.

### Exemple 2 : Amplification par PCR et détection

2.1 On utilise un appareil à thermocycle avec détection en ligne intégré pour détecter l'amplification de l'ADN spécifique, tel que l'appareil LightCycler commercialisé par la société Roche Molecular Biochemicals. 32 échantillons sont manipulés en parallèle, et 40 cycles d'amplification sont effectués à haute température (de 75°C à 100°C), les 40 cycles durant environ 45 minutes. L'amplification et la détection se déroulent dans des capillaires. Le seuil de détection est d'environ 1 organisme pathogène.
2.2 Comme alternative, on peut également utiliser le système de détection Icycler IQ, commercialisé par la société BIORAD, traitant 96 échantillons en parallèle (deux heures), pendant 40 cycles pour la détection de plus de 10 échantillons dans un flacon, durant environ 2,5 heures.

On obtient ainsi des sensibilités de l'ordre de quelques femtogrammes d'ADN/ARN total (ce qui équivaut à une cellule isolée). Le seuil de détection est d'environ 1 organisme pathogène.

## Revendications

1. Procédé d'extraction de composés ou complexes intracellulaires, tels que des acides nucléiques, à partir d'un échantillon contenant des cellules en suspension dans un liquide, **caractérisé en ce qu'**il comprend les étapes successives suivantes :
- fixation des cellules sur un support solide,
- séchage des cellules fixées sur ledit support,
- lyse des cellules sèches par micro-ondes, puis
- récupération desdits composés ou complexes intracellulaires.

2. Procédé d'extraction selon la revendication 1, **caractérisé en ce que** le liquide est de l'eau ou est constitué essentiellement d'eau.

3. Procédé d'extraction selon la revendication 1 ou 2, **caractérisé en ce que** le support solide contient au moins un matériau poreux, avantageusement adsorbant.

4. Procédé d'extraction selon l'une quelconque des revendications 1 à 3, **caractérisé en ce que** la fixation des cellules est réalisée par adsorption des cellules sur le support solide.

5. Procédé d'extraction selon l'une quelconque des revendications 1 à 4, **caractérisé en ce que** le séchage des cellules est réalisé par injection d'air ou par séchage sous vide.

6. Procédé d'extraction selon l'une quelconque des revendications précédentes, **caractérisé en ce que** la lyse des cellules par micro-ondes est effectuée à une fréquence de radiation de 1 à 15 GHz, avantageusement de 2 à 10 GHz.

7. Procédé d'extraction selon l'une quelconque des revendications précédentes, **caractérisé en ce que** la récupération des composés ou complexes intracellulaires comprend la fixation desdits composés ou complexes intracellulaires sur un support solide, suivie d'une élution desdits composés ou complexes intracellulaires, ledit support contenant avantageusement au moins un matériau poreux, de préférence adsorbant.

8. Procédé d'extraction selon la revendication 7, **caractérisé en ce que** la fixation des composés ou complexes intracellulaires est réalisée par adsorption desdits composés ou complexes intracellulaires sur le support solide.

9. Procédé d'extraction selon la revendication 7 ou 8, **caractérisé en ce que** le support solide pour la fixation des cellules et le support solide pour la fixation des composés ou complexes intracellulaires sont confondus.

10. Procédé d'extraction d'acides nucléiques de cellules en suspension dans un échantillon liquide selon l'une quelconque des revendications 1 à 9, **caractérisé en ce qu'**il comprend les étapes successives suivantes :
- adsorption des cellules sur un support solide,
- séchage des cellules adsorbées sur ledit support,
- lyse des cellules sèches par micro-ondes,
- adsorption des acides nucléiques ainsi libérés sur ledit support, puis
- élution desdits acides nucléiques.

11. Procédé d'extraction d'acides nucléiques selon la revendication 10, **caractérisé en ce qu'**il comprend en outre une étape préalable de concentration des cellules.

12. Procédé d'extraction d'acides nucléiques selon la revendication 11, **caractérisé en ce que** la concentration des cellules est réalisée par précipitation, co-précipitation ou précipitation par inclusion, avantageusement en présence de carbonates d'ions bivalents, tels que le carbonate de calcium, le carbonate de strontium, le carbonate de baryum, et leurs mélanges, et/ou la concentration des cellules est réalisée par filtration et/ou à l'aide d'un système immuno-magnétique.

13. Procédé d'extraction d'acides nucléiques selon l'une quelconque des revendications 10 à 12, **caractérisé en ce qu'**il comprend en outre une étape préalable d'ajout de calcium, avantageusement sous forme de sel.

14. Procédé d'extraction d'acides nucléiques selon l'une quelconque des revendications 10 à 13, **caractérisé en ce que** le support solide contient un matériau poreux adsorbant, avantageusement un matériau vitreux ou un composé à base de silice, tel que du verre, des fibres de verre, un oxyde de silice ou un silicate.

15. Procédé d'extraction d'acides nucléiques selon l'une quelconque des revendications 10 à 14, **caractérisé en ce que** l'adsorption des cellules sur le support solide est réalisée à pH neutre, avantageusement compris entre 7 et 7,5.

16. Procédé d'extraction d'acides nucléiques selon l'une quelconque des revendications 10 à 15, **caractérisé en ce que** suite à l'adsorption des cellules sur le support solide et préalablement au séchage des cellules adsorbées sur ledit support, ledit support est lavé à l'aide d'au moins un solvant contenant des ions monovalents, avantageusement au moins un sel de sodium, tel que le thiocyanate de sodium ou le chlorure de sodium, et/ou l'EDTA.

17. Procédé d'extraction d'acides nucléiques selon l'une quelconque des revendications 10 à 16, **caractérisé en ce que** la lyse des cellules est réalisée en effectuant un ou plusieurs cycles de chauffage, chaque cycle de chauffage consistant à chauffer les cellules adsorbées sur le support solide par micro-ondes, avantageusement à une puissance de l'ordre de 500 à 1000 Watts pendant 10 à 60 secondes, puis à laisser reposer le support solide pendant 10 à 60 secondes.

18. Procédé d'extraction d'acides nucléiques selon l'une quelconque des revendications 10 à 17, **caractérisé en ce que** lors de la lyse ou suite à la lyse des cellules, on ajoute sur le support au moins un agent chaotrope, avantageusement un sel de guanidine tel que le thiocyanate de guanidine, et/ou au moins un agent de saturation tel que le sperme de hareng, et/ou au moins un agent modificateur d'adsorption, avantageusement un alcool tel que l'éthanol.

19. Procédé d'extraction d'acides nucléiques selon l'une quelconque des revendications 10 à 18, **caractérisé en ce que** l'élution des acides nucléiques est réalisée par lavage du support solide à l'eau.

20. Procédé de détection de la présence d'organismes pathogènes dans un échantillon liquide, **caractérisé en ce qu'**il comprend les étapes successives suivantes :
- une extraction d'acides nucléiques selon le procédé tel que défini à l'une quelconque des revendications 10 à 19,
- au moins une amplification d'acides nucléiques, et
- une détection qualitative et/ou quantitative d'ADN et/ou d'ARN représentatifs de la contamination du liquide par lesdits organismes pathogènes.

21. Procédé de détection selon la revendication 20, **caractérisé en ce que** les organismes pathogènes détectés sont des bactéries, des virus, des champignons et/ou des protozoaires.

22. Procédé de détection selon la revendication 20 ou 21, **caractérisé en ce que** le volume initial d'échantillon est inférieur à 5 litres, avantageusement inférieur à 2 litres, encore plus avantageusement inférieur à 1 litre.

23. Procédé de détection selon l'une quelconque des revendications 20 à 22, **caractérisé en ce que** l'amplification d'acides nucléiques est réalisée par au moins une réaction en chaîne par polymérase avantageusement en temps réel.

24. Procédé de détection selon l'une quelconque des revendications 20 à 23, **caractérisé en ce que** la détection des organismes pathogènes est une détection en ligne, avantageusement réalisée par fluorescence, par luminescence ou par spectrométrie de masse.

25. Procédé de détection selon l'une quelconque des revendications 20 à 24, **caractérisé en ce qu'**il comprend en outre une étape préalable de traitement de l'échantillon liquide par choc thermique et/ou par ajout d'un composé chimique inducteur de gène afin d'induire l'ARN de gènes spécifiques et de détecter les organismes pathogènes viables dans ledit échantillon liquide.

26. Procédé de détection selon l'une quelconque des revendications 20 à 25, **caractérisé en ce que** l'ensemble des étapes séquentielles du procédé sont gérées de manière entièrement automatique à l'aide d'une logique de commande, telle qu'un ordinateur.

27. Dispositif d'extraction de composés ou complexes intracellulaires, tels que des acides nucléiques, à partir d'un échantillon contenant des cellules en suspension dans un liquide, **caractérisé en ce qu'**il comprend :
- au moins un moyen de fixation des cellules sur un support solide,
- au moins un moyen de séchage des cellules fixées, permettant d'éliminer le liquide extracellulaire,
- au moins un moyen de lyse des cellules sèches par micro-ondes, et
- au moins un moyen de récupération desdits composés ou complexes intracellulaires.

28. Dispositif d'extraction selon la revendication 27, **caractérisé en ce que** le liquide est de l'eau ou un liquide constitué essentiellement d'eau.

29. Dispositif d'extraction selon la revendication 27 ou 28, **caractérisé en ce que** le moyen de fixation des cellules comprend un ou plusieurs support(s) solide(s) contenant au moins un matériau poreux, avantageusement un matériau d'adsorption réversible, tel qu'un matériau vitreux ou un composé à base de silice, tel que du verre, des fibres de verre, un oxyde de silice ou un silicate.

30. Dispositif d'extraction selon l'une quelconque des revendications 27 à 29, **caractérisé en ce que** le moyen de séchage des cellules fixées comprend au moins un système d'injection d'air, avantageusement d'air comprimé, ou un système de séchage sous vide.

31. Dispositif d'extraction selon l'une quelconque des revendications 27 à 30, **caractérisé en ce que** le moyen de lyse des cellules contient au moins un magnétron.

32. Dispositif d'extraction selon l'une quelconque des revendications 27 à 31, **caractérisé en ce que** le moyen de récupération des composés ou complexes intracellulaires comprend un moyen de fixation desdits composés ou complexes intracellulaires, ledit moyen de fixation contenant un ou plusieurs support(s) solide(s) contenant au moins un matériau poreux, avantageusement un matériau d'adsorption réversible, tel qu'un matériau vitreux ou un composé à base de silice, tel que du verre, des fibres de verre, un oxyde de silice ou un silicate.

33. Dispositif d'extraction selon la revendication 32, **caractérisé en ce que** le moyen de fixation des cellules et le moyen de fixation des composés ou complexes intracellulaires sont confondus.

34. Dispositif d'extraction selon la revendication 33, **caractérisé en ce que** le(s) support(s) solide(s) pour fixer les cellules et les composés ou complexes intracellulaires est (sont) constitué(s) par des plaques de micro-titration contenant des multi-puits, avantageusement des plaques de 60, 96 ou 384 puits, chaque puit contenant au moins un matériau poreux, avantageusement adsorbant.

35. Dispositif d'extraction selon l'une quelconque des revendications 27 à 34, **caractérisé en ce qu'**il contient en outre un moyen de concentration des cellules par précipitation, co-précipitation ou précipitation par inclusion.

36. Dispositif d'extraction selon la revendication 35, **caractérisé en ce que** le moyen de concentration des cellules par précipitation, co-précipitation ou précipitation par inclusion est un réacteur comprenant un système d'introduction de l'échantillon liquide, un système d'introduction de différents réactifs, un système d'agitation, un système d'évacuation du liquide surnageant, un système d'évacuation du précipité, avantageusement placé au fond du réacteur, et un système de chauffage, avantageusement constitué d'une enveloppe chauffante placée autour du réacteur.

37. Dispositif de détection de la présence d'organismes pathogènes dans un échantillon liquide, tel que l'eau, comprenant :
- un dispositif d'extraction d'acides nucléiques de cellules en suspension dans un échantillon liquide selon l'une quelconque des revendications 27 à 36,
- un moyen d'amplification d'acides nucléiques,
- un moyen de détection qualitative et/ou quantitative d'ADN et/ou d'ARN représentatifs de la contamination du liquide par lesdits organismes pathogènes,
- des moyens de pompage, et
- des moyens de prélèvement et de distribution avantageusement automatiques, tels qu'un robot.

38. Dispositif de détection selon la revendication 37, **caractérisé en ce que** le moyen d'amplification des acides nucléiques contient au moins un appareil de réaction en chaîne par polymérase à thermocycle.

39. Dispositif de détection selon la revendication 37 ou 38, **caractérisé en ce que** le moyen de détection contient au moins un détecteur à fluorescence, avantageusement comprenant une caméra électronique.

40. Dispositif de détection selon l'une quelconque des revendications 37 à 39, **caractérisé en ce qu'**il contient en outre une logique de commande avantageusement comprenant au moins un ordinateur de type PC qui commande l'ensemble du dispositif.

41. Dispositif de détection selon la revendication 40, **caractérisé en ce que** la logique de commande est en interface avec un réseau pour transférer, de manière automatique ou sur requête, les données d'analyse relatives à la détection des organismes à un système de commande central qui est avantageusement constitué par au moins un ordinateur.

## Claims

1. Method for extracting intracellular complexes or compounds, such as nucleic acids, from a sample containing cells in suspension in a liquid, **characterized in that** it comprises the following successive steps:
- attaching the cells to a solid support,
- drying the cells attached to said support,
- lysing the dried cells by means of microwaves, then
- recovering said intracellular complexes or compounds.

2. Extraction method according to Claim 1, **characterized in that** the liquid is water or consists essentially of water.

3. Extraction method according to Claim 1 or 2, **characterized in that** the solid support contains at least one porous, advantageously adsorbent, material.

4. Extraction method according to any one of Claims 1 to 3, **characterized in that** the attaching of the cells is carried out by adsorption of the cells onto the solid support.

5. Extraction method according to any one of Claims 1 to 4, **characterized in that** the drying of the cells is carried out by air injection or by vacuum-drying.

6. Extraction method according to any one of the preceding claims, **characterized in that** the lysis of the cells by means of microwaves is carried out at a radiation frequency of from 1 to 15 GHz, advantageously from 2 to 10 GHz.

7. Extraction method according to any one of the preceding claims, **characterized in that** the recovery of the intracellular complexes or compounds comprises the attachment of said intracellular complexes or compounds to a solid support, followed by elution of said intracellular complexes or compounds, said support advantageously containing at least one porous, preferably adsorbent, material.

8. Extraction method according to Claim 7, **characterized in that** the attachment of the intracellular complexes or compounds is carried out by adsorption of said intracellular complexes or compounds onto the solid support.

9. Extraction method according to Claim 7 or 8, **characterized in that** the solid support for the attachment of the cells and the solid support for the attachment of the intracellular complexes or compounds are combined.

10. Method for extracting nucleic acids from cells in suspension in a liquid sample according to any one of Claims 1 to 9, **characterized in that** it comprises the following successive steps:
- adsorbing the cells onto a solid support,
- drying the cells adsorbed onto said support,
- lysing the dried cells by means of microwaves,
- adsorbing the nucleic acids thus released onto said support, then
- eluting said nucleic acids.

11. Method for extracting nucleic acids according to Claim 10, **characterized in that** it also comprises a prior step of concentrating the cells.

12. Method for extracting nucleic acids according to Claim 11, **characterized in that** the concentrating of the cells is carried out by precipitation, coprecipitation or inclusion precipitation, advantageously in the presence of divalent carbonate ions, such as calcium carbonate, strontium carbonate, barium carbonate, and mixtures thereof, and/or the concentrating of the cells is carried out by filtration and/or using an immunomagnetic system.

13. Method for extracting nucleic acids according to any one of Claims 10 to 12, **characterized in that** it also comprises a prior step of adding calcium, advantageously in the form of a salt.

14. Method for extracting nucleic acids according to any one of Claims 10 to 13, **characterized in that** the solid support contains an adsorbent porous material, advantageously a vitreous material or a compound based on silica, such as glass, glass fibres, a silica oxide or a silicate.

15. Method for extracting nucleic acids according to any one of Claims 10 to 14, **characterized in that** the adsorption of the cells onto the solid support is carried out at neutral pH, advantageously between 7 and 7.5.

16. Method for extracting nucleic acids according to any one of Claims 10 to 15, **characterized in that**, subsequent to the adsorption of the cells onto the solid support and prior to the drying of the cells adsorbed onto said support, said support is washed with at least one solvent containing monovalent ions, advantageously at least one sodium salt, such as sodium thiocyanate or sodium chloride, and/or EDTA.

17. Method for extracting nucleic acids according to any one of Claims 10 to 16, **characterized in that** the lysis of the cells is carried out by performing one or more heating cycles, each heating cycle consisting in heating the cells adsorbed onto the solid support by means of microwaves, advantageously at a power of the order of from 500 to 1000 watts for 10 to 60 seconds, and then in leaving the solid support to stand for 10 to 60 seconds.

18. Method for extracting nucleic acids according to any one of Claims 10 to 17, **characterized in that**, during the lysis or subsequent to the lysis of the cells, at least one chaotropic agent, advantageously a guanidine salt such as guanidine thiocyanate, and/or at least one saturation agent such as herring sperm, and/or at least one adsorption modifier, advantageously an alcohol such as ethanol, is (are) added to the support.

19. Method for extracting nucleic acids according to any one of Claims 10 to 18, **characterized in that** the elution of the nucleic acids is carried out by washing the solid support with water.

20. Method for detecting the presence of pathogenic organisms in a liquid sample, **characterized in that** it comprises the following successive steps:
- a nucleic acid extraction according to the method as defined in any one of Claims 10 to 19,
- at least one nucleic acid amplification, and
- a qualitative and/or quantitative detection of DNA and/or of RNA representative of the contamination of the liquid with said pathogenic organisms.

21. Detection method according to Claim 20, **characterized in that** the pathogenic organisms detected are bacteria, viruses, fungi and/or protozoa.

22. Detection method according to Claim 20 or 21, **characterized in that** the initial sample volume is less than 5 litres, advantageously less than 2 litres, even more advantageously less than 1 litre.

23. Detection method according to any one of Claims 20 to 22, **characterized in that** the nucleic acid amplification is carried out by means of at least one polymerase chain reaction, advantageously real time polymerase chain reaction.

24. Detection method according to any one of Claims 20 to 23, **characterized in that** the detection of the pathogenic organisms is an on-line detection, advantageously carried out by fluorescence, by luminescence or by mass spectrometry.

25. Detection method according to any one of Claims 20 to 24, **characterized in that** it also comprises a prior step of treatment of the liquid sample by heat shock and/or by the addition of a gene-inducing chemical compound in order to induce the RNA of specific genes and to detect the viable pathogenic organisms in said liquid sample.

26. Detection method according to any one of Claims 20 to 25, **characterized in that** all the sequential steps of the method are managed entirely automatically using a control logic, such as a computer.

27. Device for extracting intracellular complexes or compounds, such as nucleic acids, from a sample containing cells in suspension in a liquid, **characterized in that** it comprises:
- at least one means for attaching the cells to a solid support,
- at least one means for drying the attached cells, making it possible to eliminate the extracellular liquid,
- at least one means for lysing the dried cells by means of microwaves, and
- at least one means for recovering said intracellular complexes or compounds.

28. Extraction device according to Claim 27, **characterized in that** the liquid is water or a liquid consisting essentially of water.

29. Extraction device according to Claim 27 or 28, **characterized in that** the means for attaching the cells comprises one or more solid support(s) containing at least one porous material, advantageously a reversible adsorption material, such as a vitreous material or a compound based on silica, such as glass, glass fibres, a silica oxide or a silicate.

30. Extraction device according to any one of Claims 27 to 29, **characterized in that** the means for drying the attached cells comprises at least one air injection system, advantageously compressed air injection system, or one vacuum-drying system.

31. Extraction device according to any one of Claims 27 to 30, **characterized in that** the means for lysing the cells contains at least one magnetron.

32. Extraction device according to any one of Claims 27 to 31, **characterized in that** the means for recovering the intracellular complexes or compounds comprises a means for attaching said intracellular complexes or compounds, said attachment means containing one or more solid support(s) containing at least one porous material, advantageously a reversible adsorption material, such as a vitreous material or a compound based on silica, such as glass, glass fibres, a silica oxide or a silicate.

33. Extraction device according to Claim 32, **characterized in that** the means for attaching the cells and the means for attaching the intracellular complexes or compounds are combined.

34. Extraction device according to Claim 33, **characterized in that** the solid support(s) for attaching the cells and the intracellular complexes or compounds consist(s) of microtitration plates containing multiwells, advantageously 60-, 96- or 384-well plates, each well containing at least one porous, advantageously adsorbent, material.

35. Extraction device according to any one of Claims 27 to 34, **characterized in that** it also contains a means for concentrating the cells by precipitation, coprecipitation or inclusion precipitation.

36. Extraction device according to Claim 35, **characterized in that** the means for concentrating the cells by precipitation, coprecipitation or inclusion precipitation is a reactor comprising a system for introducing the liquid sample, a system for introducing various reagents, an agitation system, a system for evacuating the supernatant liquid, a system for evacuating the precipitate, advantageously placed at the bottom of the reactor, and a heating system, advantageously consisting of a heating jacket placed around the reactor.

37. Device for detecting the presence of pathogenic organisms in a liquid sample, such as water, comprising:
- a device for extracting nucleic acids from cells in suspension in a liquid sample according to any one of Claims 27 to 36,
- a means for amplifying nucleic acids,
- a means for qualitatively and/or quantitatively detecting DNA and/or RNA representative of the contamination of the liquid with said pathogenic organisms,
- pumping means, and
- sampling and distribution means, which are advantageously automatic, such as a robot.

38. Detection device according to Claim 37, **characterized in that** the means for amplifying nucleic acids contains at least one thermocycle polymerase chain reaction machine.

39. Detection device according to Claim 37 or 38, **characterized in that** the detection means contains at least one fluorescence detector, advantageously comprising an electronic camera.

40. Detection device according to any one of Claims 37 to 39, **characterized in that** it also contains a control logic advantageously comprising at least one PC computer which controls the entire device.

41. Detection device according to Claim 40, **characterized in that** the control logic interfaces with a network for transferring, automatically or on request, the analytical data relating to the detection of the organisms to a central control system which advantageously consists of at least one computer.

## Patentansprüche

1. Verfahren zur Extraktion von intrazellulären Verbindungen oder Komplexen, wie Nucleinsäuren, aus einer Probe, welche in einer Flüssigkeit suspendierte Zellen enthält, **dadurch gekennzeichnet, dass** es die folgenden aufeinander folgenden Schritte umfasst:
- Fixieren der Zellen auf einem festen Träger,
- Trocknen der fixierten Zellen auf dem Träger,
- Lysieren der trockenen Zellen durch Mikrowellen, dann
- Gewinnen der intrazellulären Verbindungen oder Komplexe.

2. Extraktionsverfahren nach Anspruch 1, **dadurch gekennzeichnet, dass** die Flüssigkeit Wasser ist oder im Wesentlichen aus Wasser besteht.

3. Extraktionsverfahren nach Anspruch 1 oder 2, **dadurch gekennzeichnet, dass** der feste Träger mindestens ein poröses, vorteilhaft adsorbierendes Material enthält.

4. Extraktionsverfahren nach irgendeinem der Ansprüche 1 bis 3, **dadurch gekennzeichnet, dass** das Fixieren der Zellen durch Adsorption der Zellen auf dem festen Träger verwirklicht wird.

5. Extraktionsverfahren nach irgendeinem der Ansprüche 1 bis 4, **dadurch gekennzeichnet, dass** das Trocknen der Zellen durch Einspritzen von Luft oder durch Trocknen unter Vakuum vorgenommen wird.

6. Extraktionsverfahren nach irgendeinem der vorangehenden Ansprüche, **dadurch gekennzeichnet, dass** das Lysieren der Zellen durch Mikrowellen bei einer Strahlungsfrequenz von 1 bis 15 GHz, vorteilhaft 2 bis 10 GHz, bewirkt wird.

7. Extraktionsverfahren nach irgendeinem der vorangehenden Ansprüche, **dadurch gekennzeichnet, dass** das Gewinnen der intrazellulären Verbindungen oder Komplexe das Fixieren der intrazellulären Verbindungen oder Komplexe auf einem festen Träger, gefolgt von einer Elution der intrazellulären Verbindungen oder Komplexe, umfasst, wobei der Träger vorteilhaft mindestens ein poröses, bevorzugt adsorbierendes Material enthält.

8. Extraktionsverfahren nach Anspruch 7, **dadurch gekennzeichnet, dass** das Fixieren der intrazellulären Verbindungen oder Komplexe durch Adsorption der intrazellulären Verbindungen oder Komplexe auf dem festen Träger verwirklicht wird.

9. Extraktionsverfahren nach Anspruch 7 oder 8, **dadurch gekennzeichnet, dass** der feste Träger für die Fixierung der Zellen und der feste Träger für die Fixierung der intrazellulären Verbindungen oder Komplexe vereinigt sind.

10. Verfahren zur Extraktion von Nucleinsäuren aus in einer flüssigen Probe suspendierten Zellen nach irgendeinem der Ansprüche 1 bis 9, **dadurch gekennzeichnet, dass** es die folgenden aufeinander folgenden Schritte umfasst:
- Adsorbieren der Zellen auf einem festen Träger,
- Trocknen der adsorbierten Zellen auf dem Träger,
- Lysieren der trockenen Zellen durch Mikrowellen,
- Adsorbieren von so freigesetzten Nucleinsäuren auf dem Träger, dann
- Eluieren der Nucleinsäuren.

11. Verfahren zur Extraktion von Nucleinsäuren nach Anspruch 10, **dadurch gekennzeichnet, dass** es darüber hinaus einen vorgeschalteten Schritt des Konzentrierens der Zellen umfasst.

12. Verfahren zur Extraktion von Nucleinsäuren nach Anspruch 11, **dadurch gekennzeichnet, dass** das Konzentrieren der Zellen durch Präzipitation, Copräzipitation oder Präzipitation durch Einschluss vorteilhaft in Anwesenheit von zweiwertigen Carbonat-Ionen, wie Calciumcarbonat, Strontiumcarbonat, Bariumcarbonat und deren Mischungen, vorgenommen wird und/oder das Konzentrieren der Zellen durch Filtration und/oder mit Hilfe eines immunmagnetischen Systems vorgenommen wird.

13. Verfahren zur Extraktion von Nucleinsäuren nach irgendeinem der Ansprüche 10 bis 12, **dadurch gekennzeichnet, dass** es darüber hinaus einen vorgeschalteten Schritt der Zugabe von Calcium, vorteilhaft in Salzform, umfasst.

14. Verfahren zur Extraktion von Nucleinsäuren nach irgendeinem der Ansprüche 10 bis 13, **dadurch gekennzeichnet, dass** der feste Träger ein poröses adsorbierendes Material, vorteilhaft ein glasartiges Material oder eine Verbindung auf Siliciumdioxid-Basis, wie Glas, Glasfasern, Oxid von Silicium oder ein Silicat, enthält.

15. Verfahren zur Extraktion von Nucleinsäuren nach irgendeinem der Ansprüche 10 bis 14, **dadurch gekennzeichnet, dass** die Adsorption der Zellen auf dem festen Träger bei neutralem pH, vorteilhaft zwischen 7 und 7,5 einschließlich, bewerkstelligt wird.

16. Verfahren zur Extraktion von Nucleinsäuren nach irgendeinem der Ansprüche 10 bis 15, **dadurch gekennzeichnet, dass** nach der Adsorption der Zellen auf dem festen Träger und vor dem Trocknen der adsorbierten Zellen auf dem Träger der Träger mit mindestens einem Lösungsmittel gewaschen wird, das einwertige Ionen enthält, vorteilhaft mindestens ein Natriumsalz, wie Natriumthiocyanat oder Natriumchlorid, und/oder EDTA.

17. Verfahren zur Extraktion von Nucleinsäuren nach irgendeinem der Ansprüche 10 bis 16, **dadurch gekennzeichnet, dass** die Lyse der Zellen bewirkt wird, indem-man einen oder mehrere Erwärmungszyklen bewirkt, wobei jeder Erwärmungszyklus darin besteht, die auf dem festen Träger adsorbierten Zellen durch Mikrowellen, vorteilhaft bei einer Leistung in der Größenordnung von 500 bis 1000 Watt, 10 bis 60 Sekunden lang zu erwärmen, dann den festen Träger 10 bis 60 Sekunden lang ruhen zu lassen.

18. Verfahren zur Extraktion von Nucleinsäuren nach irgendeinem der Ansprüche 10 bis 17, **dadurch gekennzeichnet, dass** man während der Lyse oder nach der Lyse der Zellen mindestens ein chaotropes Mittel, vorteilhaft ein Guanidinsalz, wie Guanidinthiocyanat, und/oder mindestens ein Sättigungsmittel, wie Heringssperma, und/oder mindestens ein Adsorptionsmodifikationsmittel, vorteilhaft einen Alkohol, wie Ethanol, auf den Träger gibt.

19. Verfahren zur Extraktion von Nucleinsäuren nach irgendeinem der Ansprüche 10 bis 18, **dadurch gekennzeichnet, dass** die Elution der Nucleinsäuren durch Waschen des festen Trägers mit Wasser durchgeführt wird.

20. Verfahren zum Nachweis der Anwesenheit von pathogenen Organismen in einer flüssigen Probe, **dadurch gekennzeichnet, dass** es die folgenden aufeinander folgenden Schritte umfasst:
- eine Extraktion von Nucleinsäuren nach dem Verfahren, wie in irgendeinem der Ansprüche 10 bis 19 definiert,
- mindestens eine Amplifikation von Nucleinsäuren und
- einen qualitativen und/oder quantitativen Nachweis von DNA und/oder RNA, die für die Kontamination der Flüssigkeit durch die pathogenen Organismen repräsentativ sind.

21. Nachweisverfahren nach Anspruch 20, **dadurch gekennzeichnet, dass** die nachgewiesenen pathogenen Organismen Bakterien, Viren, Pilze und/oder Protozoen sind.

22. Nachweisverfahren nach Anspruch 20 oder 21, **dadurch gekennzeichnet, dass** das anfängliche Probenvolumen unter 5 Litern, vorteilhaft unter 2 Litern, noch vorteilhafter unter 1 Liter liegt.

23. Nachweisverfahren nach irgendeinem der Ansprüche 20 bis 22, **dadurch gekennzeichnet, dass** die Amplifikation von Nucleinsäuren durch mindestens eine Polymerasekettenreaktion vorteilhaft in Echtzeit durchgeführt wird.

24. Nachweisverfahren nach irgendeinem der Ansprüche 20 bis 23, **dadurch gekennzeichnet, dass** der Nachweis der pathogenen Organismen ein in-line-Nachweis ist, der vorteilhaft durch Fluoreszenz, Lumineszenz oder Massenspektrometrie durchgeführt wird.

25. Nachweisverfahren nach irgendeinem der Ansprüche 20 bis 24, **dadurch gekennzeichnet, dass** es weiter einen vorgeschalteten Schritt der Behandlung der flüssigen Probe durch Wärmeschock und/oder durch Zugabe einer chemischen Gen-induzierenden Verbindung umfasst, um die RNA von spezifischen Genen zu induzieren und die lebensfähigen pathogenen Organismen in der flüssigen Probe nachzuweisen.

26. Nachweisverfahren nach irgendeinem der Ansprüche 20 bis 25, **dadurch gekennzeichnet, dass** alle aufeinander folgenden Schritte des Verfahrens auf vollständig automatische Weise mit Hilfe einer logischen Steuerung, wie eines Computers, durchgeführt werden.

27. Vorrichtung zur Extraktion von intrazellulären Verbindungen oder Komplexen, wie Nucleinsäuren, aus einer Probe, welche in einer Flüssigkeit suspendierte Zellen enthält, **dadurch gekennzeichnet, dass** sie umfasst:
- mindestens ein Mittel zum Fixieren von Zellen auf einem festen Träger,
- mindestens ein Mittel zum Trocknen der fixierten Zellen, welches es ermöglicht, die extrazelluläre Flüssigkeit zu beseitigen,
- mindestens ein Mittel zum Lysieren der trockenen Zellen durch Mikrowellen und
- mindestens ein Mittel zum Gewinnen der intrazellulären Verbindungen oder Komplexe.

28. Extraktionsvorrichtung nach Anspruch 27, **dadurch gekennzeichnet, dass** die Flüssigkeit Wasser oder eine im Wesentlichen aus Wasser bestehende Flüssigkeit ist.

29. Extraktionsvorrichtung nach Anspruch 27 oder 28, **dadurch gekennzeichnet, dass** das Mittel zum Fixieren der Zellen einen oder mehrere feste Träger umfasst, die mindestens ein poröses Material, vorteilhaft ein Material zur reversiblen Adsorption, wie ein glasartiges Material oder eine Verbindung auf Siliciumdioxid-Basis, wie Glas, Glasfasern, ein Oxid von Silicium oder ein Silicat, enthalten.

30. Extraktionsvorrichtung nach irgendeinem der Ansprüche 27 bis 29, **dadurch gekennzeichnet, dass** das Mittel zur Trocknung der fixierten Zellen mindestens ein Lufteinspritzungs-System, vorteilhaft für komprimierte Luft, oder ein System zur Trocknung unter Vakuum umfasst.

31. Extraktionsvorrichtung nach irgendeinem der Ansprüche 27 bis 30, **dadurch gekennzeichnet, dass** das Mittel zur Lyse der Zellen mindestens ein Magnetron enthält.

32. Extraktionsvorrichtung nach irgendeinem der Ansprüche 27 bis 31, **dadurch gekennzeichnet, dass** das Mittel zum Gewinnen der intrazellulären Verbindungen oder Komplexe ein Mittel zum Fixieren der intrazellulären Verbindungen oder Komplexe umfasst, wobei das Fixierungsmittel einen oder mehrere feste Träger enthält, die mindestens ein poröses Material, vorteilhaft ein Material zur reversiblen Adsorption, wie ein glasartiges Material oder eine Verbindung auf Siliciumdioxid-Basis, wie Glas, Glasfasern, ein Oxid von Silicium oder ein Silicat, enthalten.

33. Extraktionsvorrichtung nach Anspruch 32, **dadurch gekennzeichnet, dass** das Mittel zum Fixieren der Zellen und das Mittel zum Fixieren der intrazellulären Verbindungen und Komplexe vereinigt sind.

34. Extraktionsvorrichtung nach Anspruch 33, **dadurch gekennzeichnet, dass** der oder die feste(n) Träger zum Fixieren der Zellen und der intrazellulären Verbindungen oder Komplexe aus Mikrotiterplatten besteht/bestehen, die viele Vertiefungen enthalten, vorteilhaft Platten mit 60, 96 oder 384 Vertiefungen, wobei jede Vertiefung mindestens ein poröses, vorteilhafterweise adsorbierendes Material enthält.

35. Extraktionsvorrichtung nach irgendeinem der Ansprüche 27 bis 34, **dadurch gekennzeichnet, dass** sie weiter ein Mittel zur Konzentration der Zellen durch Präzipitation, Copräzipitation oder Präzipitation durch Einschluss enthält.

36. Extraktionsvorrichtung nach Anspruch 35, **dadurch gekennzeichnet, dass** das Mittel zur Konzentration der Zellen durch Präzipitation, Copräzipitation oder Präzipitation durch Einschluss ein Reaktor ist, der ein System zur Einführung der flüssigen Probe, ein System zur Einführung von verschiedenen Reagenzien, ein System zum Umwälzen, ein System zur Entfernung von flüssigem Überstand, ein System zur Entfernung von Niederschlag, das vorteilhaft am Boden des Reaktors angeordnet ist, und ein Heizsystem umfasst, das vorteilhaft aus einem Heizmantel besteht, der um den Reaktor herum angeordnet ist.

37. Vorrichtung zum Nachweis der Anwesenheit von pathogenen Organismen in einer flüssigen Probe, wie Wasser, umfassend:
- eine Vorrichtung zur Extraktion von Nucleinsäuren aus in einer flüssigen Probe suspendierten Zellen nach irgendeinem der Ansprüche 27 bis 36,
- ein Mittel zur Amplifikation von Nucleinsäuren,
- ein Mittel zum qualitativen und/oder quantitativen Nachweis von DNA und/oder RNA, die für die Kontamination der Flüssigkeit durch die pathogenen Organismen repräsentativ sind,
- Mittel zum Pumpen und
- Mittel zur vorteilhafterweise automatischen Entnahme und Verteilung, wie einen Roboter.

38. Nachweisvorrichtung nach Anspruch 37, **dadurch gekennzeichnet, dass** das Mittel zur Amplifikation von Nucleinsäuren mindestens eine Vorrichtung für die thermozyklische Polymerasekettenreaktion enthält.

39. Nachweisvorrichtung nach Anspruch 37 oder 38, **dadurch gekennzeichnet, dass** das Mittel zum Nachweis mindestens einen Fluoreszenzdetektor enthält, der vorteilhaft eine elektronische Kamera umfasst.

40. Nachweisvorrichtung nach irgendeinem der Ansprüche 37 bis 39, **dadurch gekennzeichnet, dass** sie weiter eine Steuerungslogik enthält, die vorteilhaft mindestens einen Computer vom PC-Typ umfasst, der die gesamte Vorrichtung steuert.

41. Nachweisvorrichtung nach Anspruch 40, **dadurch gekennzeichnet, dass** die Steuerungslogik eine Schnittstelle mit einem Netz aufweist, um die Analysedaten mit Bezug auf den Nachweis der Organismen automatisch oder auf Anfrage zu einem zentralen Steuerungssystem zu übermitteln, das vorteilhaft aus mindestens einem Computer besteht.
